# EUROPEAN PATENT SPECIFICATION

(11) **EP 1 113 839 B1**
(45) Date of publication and mention of the grant of the patent: **03.11.2004**
(21) Application number: 99942183.7
(22) Date of filing: 17.08.1999
(51) Int. Cl.: A61M 37/00

(54) **VARIABLE OUTPUT APPLICATOR FOR MULTI-COMPONENT BIOLOGIC FLUID AGENTS**
AUFTRAGEVORRICHTUNG MIT VARIABLER ABGABE FÜR BIOLOGISCHE WIRKSTOFFE DIE AUS MEHREREN FLÜSSIGEN KOMPONENTEN BESTEHEN
APPLICATEUR A DEBIT VARIABLE POUR AGENTS FLUIDES BIOLOGIQUES A CONSTITUANTS MULTIPLES

(30) Priority: 17.08.1998 US 96816 P
(43) Date of publication of application: 11.07.2001
(73) Proprietor: BAXTER INTERNATIONAL INC., Deerfield, Illinois 60015 (US)
(72) Inventor: EPSTEIN, Gordon, Howard, Fremont, CA 94539 (US); LEVINSON, Mitchell, E., Pleasanton, CA 94588 (US); SPERO, Richard, K., Brentwood, CA 94513 (US)
(74) Representative: Dee, Ian Mark
(86) International application number: PCT/US1999/018496
(87) International publication number: WO 2000/009074

(56) References cited:
- EP-A- 0 538 174
- WO-A-98/02098
- US-A- 4 040 420
- US-A- 4 735 616
- US-A- 5 240 146
- US-A- 5 286 258
- US-A- 5 477 987
- US-A- 5 656 035
- US-A- 5 674 394

## Description

### BACKGROUND OF THE INVENTION

### 1. Field of the Invention

The present invention relates to an applicator for applying a biologic fluid agent comprising multiple fluid components to a work surface, and is particularly, although not exclusively, useful for applying biologic sealants, or other biologic fluid agents, to biological tissue to effect hemostasis, close wounds, apply skin grafts or achieve other desirable therapeutic results. More particularly, the invention relates to application of a two-component tissue sealant agent, one component of which comprises a polymerizable biological sealant agent, for example fibrinogen, and the other component of which comprises a biologically acceptable polymerization catalyst or activator agent, for example, thrombin. Other polymerization and activation agents may be used, as is understood by those skilled in the art. Though inspired by the problems of users in the surgical arts, the invention is generally useful in other arts requiring the application of multi-component sealants, adhesives, glues or other fluid agents, including arts where non-biologic agents are employed.

### 2. Description of Related Art

Use of tissue sealants, with or without additional biologically useful materials, is an important energing surgical technique, well suited to the operating room or field environments such as the doctor's office or mobile medical units. Preferred sealants include fibrin sealants which are formed from blood plasma components and comprise, on the one hand, a polymerization first component containing fibrinogen and Factor XIII, and on the other hand a catalytic second component which usually includes thrombin, and calcium ions. The fibrinogen polymerizes and cross-links to form a solid fibrin clot when the two components are mixed. The necessary additional factors to simulate relevant portions of the natural blood coagulation cascade are suitably distributed between the fibrinogen and thrombin components, as is known in the art. The sealant's "setup" or setting time, which can be considered as the time from mixing to gelling, is related to the concentration of the activator, thrombin. Higher thrombin levels yield gelling, is related to the concentration of the activator, thrombin. Higher thrombin levels yield faster setting times, which may be as short as 10 seconds. The ultimate hardness of the cured sealant is related to the concentration of the polymerizable agent, fibrinogen, higher concentrations of which yield harder products. If desired, high levels of protection against transmission of infections, or induction of immunological reactions, can be assured by using an autologous or single-donor source for one or, preferably, both components.

Such sealants, known as "fibrin sealants", are highly effective, are biologically degraded without residue and may promote wound healing. Fibrin sealants are far superior to mechanical closure means such as staples, sutures and the like, providing, in most cases, superior closure, and eliminating the need for follow-up visits to remove the closure means. Synthetic analogs to natural sealants, such as cyanoacrylate adhesives may not be biologically degraded and cannot promote wound healing.

Antanavich et al. United States Patent number 5,585,007, provides an extensive discussion of the literature relating to fibrinogen sealant preparation (column 1, line 20 to column 4, line 62) and applicators (column 4, line 62 to column 5, line 14), as well as a bibliography, (columns 6-10). Read in conjunction with the cited literature, Antanavich is a helpful guide to the teachings of prior workers in the field.

A popular, manually operable applicator for two-component sealants, for example, fibrin sealants, employs a dual syringe construction wherein two syringes, connected by a yoke, each provide a reservoir for one of the components. In many prior devices, the sealant components are discharged in separate drops or streams and mixed externally of the applicator. Such applicators are similar, in principle, to dual-syringe epoxy glue applicators commonly available in hardware stores for household and handyman use. Achieving effective mixing externally of the applicator is problematic.

Such droplet applicator devices which dispense sealant or sealant agents in small quantities from one or more dispensing tubes or cannulas, at low pressure, are useful primarily for localized tissue treatment to close wounds, secure grafts and the like. More widespread applications, such as treating diffuse oozing and coating of substantial tissue areas with sealant to prevent fluid leaks, are better effected by spray applicators. To this end, a number of two-component sealant spray applicators have been proposed with varying degrees of complexity and success.

Capozzi United States patents numbers 4,978,336 and 5,116,315 constructions of dual syringe applicator which teach internal mixing of two sealant components and discharge of the mixed components through a needle, or a spray head. To solve the problem of internal clogging that occurs when the sealant sets up, Capozzi teaches that the needle assembly or spray assembly, where the mixing occurs, should be changed. Changing such assemblies in the field, which as taught by Capozzi are detachably locked to the applicator can be a significant or even serious inconvenience or interruption to a surgical procedure.

Epstein United States patent No. 5, 266,877, teaches various constructions of dual syringe applicator wherein the fluid components are also mixed internally.

The possibility of retrograde clearing of the mixed fluids pathway within the applicator, using suction, is also disclosed. A biologic fluid applicator having particular, but not exclusive, utility for applying a sealant is also disclosed. The applicator is provided with suitable suction conduits and valving to apply suction to a work surface, to prepare the surface for the application of sealant, for example by removing fluids or other detritus therefrom. The valving is operable to effect retrograde clearing of a sealant dispensing pathway. Enhanced mixing results from internal mixing, and problems of fouling by deposited solids are avoided. Additional clearing techniques are taught in Levinson provisional patent application number 60/086,208. Epstein application number 60/083,854 teaches a spray applicator for dispensing mixed fluid components which can also employ clog clearing techniques.

Typically, prior fibrin sealant applicators have provided for the respective fibrin sealant components to be driven from respective dual reservoirs in a manner providing a constant output of sealant of a single predetermined character in mixed or unmixed form. The character of the sealant is predetermined when the applicator is loaded with sealant components, and cannot subsequently be changed or varied by the operator whose control options are limited to determining where and at what rate the sealant is dispensed.

Though such constant output devices are satisfactory for many purposes, it would be desirable obtain a different output.

US-A-4,735,616 discloses an arrangement for applying a tissue adhesive. The arrangement comprises a plurality of syringe bodies ending in joining pieces and commonly actuable by pistons to contain the components to be applied. A connecting head is attachable to the joining pieces of the syringe bodies, which includes a separate conveying channel for each of the components to be applied and optionally for a medical propellant. EP-A-0,538,174 discloses a manually operated dispensing device for dispensing two or more fluids which are contained within respective syringes which are received and supported by the dispensing device.

US-A-5,286,258 discloses a variable ratio multipharmaceutical delivery system, typically in the form of a syringe, suitable for the simultaneous delivery of two or more mixed flowable pharmaceuticals in selected amounts and proportions.

Holm U.S. patent 5,520,658 discloses a dual syringe fluid dispensing device for dispensing tissue glue, drugs and the like from syringes in 80-81 which have unequal diameters. One object is apparently to avoid wasting more expensive or more toxic liquid by first mounting the larger syringe 80 containing the less expensive or toxic liquid in the dispensing device and then adjusting a propeller 70 (which may be considered as a yoke) to mount syringe 81, containing the more expensive or more toxic liquid, in the dispensing device. As propeller 70 is adjusted, less expensive fluid may be lost from syringe 80.

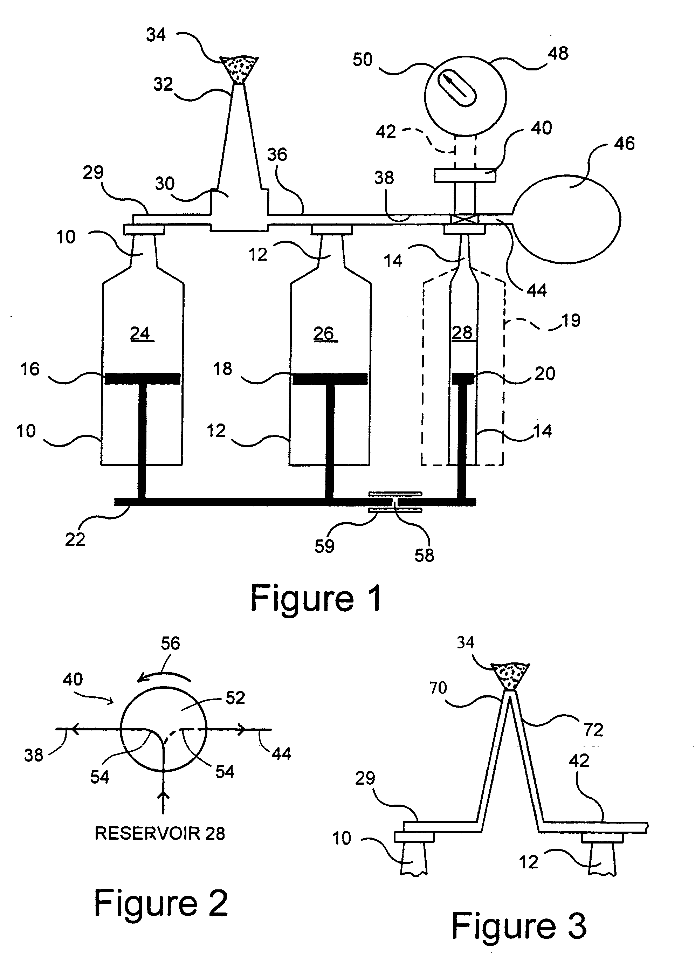

The embodiment of Figs 3-4 relates to a dispensing device which provides separate drives or transmissions for discharging the individual syringes 80-81, by squeezing trigger 60. Syringe 80 is discharged by a plunger 90 which is driven by propeller 70' moving in tandem with rack 42'. Idler pin 62 mounted on the trigger shaft 64 rotates to move toothed rack 42" forwardly, when trigger 60 is squeezed. Idler pin 62" is mounted on a sliding pin 68 which can be manipulated to vary the length of the pin, "and consequently (provide) an alteration of the ratio of transmission", see column 10, lines 60-61. Such "alteration" of the length of pin 62" is severely limited by the geometry of the device, especially the clearance between toothed rack 42" and shaft 64, and the need to maintain a sufficiently acute angle of pin 62" to generate an adequate driving force.

A further significant drawback of Holm's dispensing device is that at least at lower ratios, the output is not consistent throughout the stroke. Thus, when lever 68 is at a lower position, idler pin 62" may disengage from rack 42", terminating its advance before the travel of companion, rack 42' is completed. Consequently, output of fluid from syringe 81 terminates, while that of companion syringe 80 continues. In many cases, such inconsistent outputs are unacceptable. For example, where a two-component fibrin sealant is dispensed during a surgical procedure, instances where a solitary non-functional sealant is dispensed may occur, leading to failures of the tissue seal.

Thus it would be desirable to provide a multi-component fluid applicator which permits selection of the proportion of fluids dispensed and can consistently dispense the selected proportion.

Furthermore, Holm's variable ratio transmission as comprised by sliding pin 68 and the engagement of idler pin 62" between protruding parts 44" and 46" appears capable of only small alterations in ratio, when engaged, such as may provide for minor adjustments of degree. Such limited variability is believed inadequate to provide different outputs displaying a useful difference in kind, for example as between a fast-setting fibrinogen adhesive and a slow-setting one. Thus it would furthermore be desirable to provide an applicator selectable for outputs that are different in kind.

It would also be desirable for the surgeon to have the option of adding a controlled concentration of an auxiliary agent such as an antibiotic, chemotherapeutic or the like, to a dual-component output such as a sealant, in a manner not requiring reloading of the applicator. Such options should preferably be quickly and conveniently available in a manner compatible with the exigencies of a surgical environment.

### BRIEF SUMMARY OF THE INVENTION

According to the present invention, there is provided a variable output handheld fluid agent applicator according to claim 1.

Preferably, a desired agent output can be selected from an available range of agent outputs of different characteristics, without changing, or directly manipulating or refilling the fluid component sources. The differences in character may be quantitative, or qualitative and may comprise variations in the concentration of one of the fluid components, e.g. in the case of a biologic sealant agent, thrombin or fibrinogen, variations in the character of one or more of the fluid components, or may provide for the addition of a third component to the agent composition to enhance or supplement the properties of the composition.

The selection can be made by a manually operable actuator, or control knob, coupled to a switching device component of the agent delivery system. The switching device is operable to switch a desired agent output to the dispensing tip. Preferably also, the applicator is designed with a body supportable and manipulable by one hand of a user so that the applicator tip to which the agent output is delivered can be accurately juxtaposed to a work surface and can be effectively manipulated while agent is dispensed through the tip. For this purpose, a relatively short tip is preferred, for example a tip extending no more than a few centimeters from a user's hand. Such a tip length is preferably less than 6 cm and more preferably is less than 3 cm. In preferred embodiments, the tip is no more than one third, preferably one fifth, as long as the overall body of the applicator without the tip for compactness, manipulability and to reduce clogging where sealant components are dispensed and mixed internally.

By choice of a suitable differential drive for the delivery system, for example a lost motion differential drive, a selectable ratio gear drive or a constantly engaged adjustable lever drive, it is possible for the applicator to provide a consistent multi-fluid output of user selected character.

Preferred embodiments of applicator are manually actuated and powered, as well as being intended to be hand held in use. However, it is also contemplated that an appropriate power drive, for example a compact, low weight power assistance module carried by the applicator, such as a rechargeable battery powered motor, could be used to provide a fluid dispensing force, the force acting with durations precisely controllable by the operator.

Preferably also, the differential drive has sufficient range to provide an applicator selectable for outputs that are different in kind. To this end, a desirable range of drive ratios preferably includes ratios that provide at least a 50 percent difference in the flow rate of one of the fluid components, comparing the maximum and minimum flow rates of the one component with respect to a fixed flow rate of at least one other component. More preferably the range of variation in the one component, while a fixed output is provided from the other, is at least two to one, maximum to minimum, with larger ratios of three or more to one, being contemplated by the invention.

The particular agent outputs available to the operator will depend upon the available fluid component sources and the functional characteristics of the agent delivery system. In a simple embodiment of variable output agent applicator, the applicator comprises three fluid component reservoirs, or, possibly, connections to three external fluid component sources, and the switching device selects between two combinations of the three available fluid components, to provide the desired agent output. For example, to dispense a fibrin sealant agent, the three fluid components could comprise a fibrinogen component, a low concentration thrombin component and a high concentration thrombin component, and the switching device could selectively connect the fibrinogen component and either the low or the high concentration thrombin component, to the applicator tip, to provide the agent output. This arrangement gives the surgeon a convenient, easily enabled, choice between a slow setting and a fast setting fibrin sealant. In one embodiment of the inventive dispensing system, the three reservoirs are discharged by plungers driven in harness by a single yoke and the output of the unused reservoir is switched to waste, by the switching device, or evacuated.

In another embodiment of the invention, the applicator comprises three, or possibly more, fluid component reservoirs, and the agent delivery system comprises multiple fluid discharge members, one for each reservoir, each fluid discharge member being movable to discharge a controlled volume of a respective fluid component from its reservoir, when a suitable drive force is applied to the discharge member. Depending upon the construction of the reservoir, the drive members can be disposed within the reservoirs and can engage the fluid components directly, being constructed for example as plungers, or each drive member can engage an external wall of the reservoir and deform or displace it, being for example, a lever acting on a bladder.

It is convenient, and customary, for the drive members, e.g. the plungers of a dual syringe, to be coupled together, by a coupling member, for example a yoke, for movement in unison, whereby a single actuating movement applied by the user effects a measured displacement of each drive member simultaneously, and each drive member advances at the same speed. Pursuant to an important group of embodiments of the invention, the conventional coupling member is modified to provide, or is replaced by, a differential drive system. The differential drive system causes at least one of the drive members to advance (or, possibly, to be retracted) at a different speed with respect to one of the other drive members, so that it has a different displacement in a given time interval. Such a differential drive permits different controlled volumes to be simultaneously discharged from reservoirs of uniform cross-section, by advancing one of the drive members at a different speed from at least one other.

Preferably the differential drive is selectable so that the operator can vary the speed of least one of the plungers and thus vary the rate of dispensing of at least one of the fluid components. Preferably also, the applicator includes a switching device to enable the user to make the selection. Such a differential drive provides the user with quantitative control over the constitution of the agent output. However, it is conceivable that the differential drives described herein could be used to discharge one reservoir at a different, but fixed, speed in relation to another to provide a particularly desired, but non-variable agent output.

A desired drive relationship between the movement of one plunger and that of another can be obtained by a variety of mechanisms employing, for example, gears, pivoted levers, sliding wedges, or electrical, or pneumatic power, as is further described herein, or as will be known to, or apparent to, those skilled in the art.

Another embodiment of the invention providing a differential output with a constant drive delivery system, employs interchangeable reservoirs having different internal cross-sectional areas, for example larger and smaller diameters, which accordingly provide different fluid component flow rates when discharged by a plunger at a given speed. With advantage, such reservoirs, which may be syringe-like cartridges received into suitable bays within the applicator, can have the same effective external cross-sectional area to provide a measure of modularity and simplicity in the design of an applicator capable of providing a variety of different agent outputs. Though useful, such a construction does impose on the user the need to change the cartridges in order to vary the output of the applicator.

In a further alternative embodiment of agent delivery system, three plunger-equipped fluid component reservoirs, or syringes, have their plungers coupled together to move in unison. The flows of fluid components from two of the reservoirs are completely directed to the agent output, while the fluid component flow from the third reservoir is applied to a fluid switching device. In this case the switching device acts as a two-way valve, selectively directing, or proportioning, the third fluid component between the agent output and waste.

Thus, as a result of the invention, it is no longer necessary to change syringes, or to reload the applicator with one or more fluid components, or to re-connect a different fluid component source to the applicator, or perform some other cumbersome procedure that could interrupt a surgeon's workflow, in order to vary the qualitative or quantitative characteristics of the agent output. Rather, the surgeon, or other user is enabled to select between different agent outputs, according to the patient's needs, simply by dialing, or otherwise operating, an externally accessible manual control on the applicator. In a preferred construction, the agent reservoirs and delivery system are contained within a housing of ergonomic construction facilitating manipulation of the applicator and which preferably also provides an esthetic appearance.

Where a mixing chamber is employed in the applicator to provide a mixed agent output, and the applicator is employed for dispensing a sealant agent, pressure isolation valves, or flow restricters, can also be provided, if desired, on the one hand between the third reservoir and the mixing volume, and on the other hand between the third reservoir and waste, to control back pressure induced perturbations of flow caused by clogging or residual solids in the mixing volume. Also, if desired, a pressure-sensitive visual indicator can be provided to flag the user when clogging occurs in the mixed agent pathway prompting the user to take appropriate clearing measures.

In one preferred embodiment, the range of agent outputs available for selection by the user comprises a small number of selections such as two, three or four. Such an embodiment is particularly suitable for dispensing sealants with different thrombin concentrations to give the user a choice between fast and slow setting times, and optionally, one or two or more intermediate settings. An alternative preferred embodiment provides a continuous range of available agent outputs from which a desired output may be selected. The particular agent characteristic that varies will depend upon the construction of the agent delivery system in the applicator, and the nature of the agents selected for delivery.

Where the applicator provides a third fluid agent in addition to a polymerizable agent and a polymerization catalyst, the concentration of the third agent in the output may be selected from a continuous range of concentrations, providing the user with precise control over, for example, the concentration of a therapeutic agent added to a fibrin sealant.

In another preferred embodiment of the invention, the fluid component sources comprise two conventional sources of a liquid polymerizable agent, for example fibrinogen, and a liquid polymerization activator, for example thrombin, and additionally comprise a third and novel component source being a diluent for the activator. The diluent can be mixed, or delivered, by the agent delivery system, in varying proportions with the activator to provide agent outputs with different activity levels. Alternatively, the diluent can be delivered as a component of the agent output in a constant proportion with the polymerizable agent and varying amounts of the polymerization activator can be mixed with, or delivered with the diluent or with both the diluent and the polymerization agent, to provide a selectable range of setting times of the agent output.

A further alternative provides, in addition to the conventional sources of polymerization agent and of activator at a predetermined concentration, one or more additional sources of activator agent, each activator source having its own activator concentration to serve a purpose different from the other, whereby different concentrations of activator and thence different setup times can be selected by selecting the appropriate activator source. Thus, for a fibrin sealant, the invention provides a method and device which can dispense fibrin sealant with a selectable concentration of thrombin.

A method of applying an agent to a work surface from a hand-held dispenser is also described, the method comprising:
a) manually operating a selector on the dispenser to select a desired agent output from a range of available agent outputs of different agent characters; and
b) dispensing the selected agent output from the dispenser to the work surface.

The method can be practiced employing various ranges and types of agent outputs, as described herein in connection with the disclosed inventive applicator. Similarly, a number of different ways of delivering a desired combination of fluid components from multiple agent sources to make a particular range of agent outputs available to the user, will be apparent from the disclosure herein. For example, three fluid components can be dispensed from respective reservoirs at constant rates, and two of the components can be interchangeably switched, by a manually operated switching device, between the agent output and waste, providing a choice of agents with which the first component can be mixed by simply flipping a manual control.

Alternatively, the respective rate of discharge of least one of the fluid components versus another, may be varied in response to the user's controlling input to provide a quantitative variation in the character of the agent output.

In general terms, by providing a third liquid agent source, additional to the conventional polymerization agent and activator, and providing the possibility of varying the extent to which at least one of the liquid agents is included in the agent output, in a manually selectable manner, the invention enables a wide range of new biologicand other application features to be provided.

For example, the third liquid could comprise a conditioning or therapeutic agent, which therapeutic or conditioning agent could be mixed with one of the other agents in a concentration which is readily manually selectable by the surgeon, or other user, at the point of application and at the time of application.

Preferred embodiments of the invention comprise droplet applicators with internal or external fluid component mixing. However, the invention also contemplates providing a variable output spray applicator by dispensing two (or possibly more) overlapping spray patterns for mixing on a target work surface and varying the character, concentration or proportion of one of the spray components versus the other(s). A still more preferable variable output spray applicator, pursuant to the invention, employs gas entrainment and dispersal of a dispensed droplet stream (which could be a more or less continuous flow), of mixed components, for example, as disclosed in Epstein patent application number 60/083,854.

### BRIEF DESCRIPTION OF THE SEVERAL VIEWS OF THE DRAWING

One or more embodiments of the invention and of making and using the invention, as well as the best mode contemplated of carrying out the invention, are described in detail below, by way of example, with reference to the accompanying drawings, in which:-
- Figure 1: is a schematic plan view of one embodiment of an agent applicator according to the invention employing three fluid component reservoirs which can be used, inter alia, for applying a fibrin sealant during surgical procedures;
- Figure 2: is a schematic view of a two-way valve for use in the applicator shown in Figure 1;
- Figure 3: is a schematic plan view of a modification of the embodiment of Figures 1 and 2;
- Figure 4A: is a schematic plan view of an agent applicator according to the invention employing three fluid component reservoirs and a differential drive system to vary the agent output from one or more of the reservoirs;
- Figure 4B: is a partial view of the differential drive system of the applicator shown in Figure 4A;
- Figure 4C: is a partial view of another differential drive system of the applicator shown in Figure 4A;
- Figure 5: is a schematic partial side elevation of a still further embodiment of an agent applicator according to the invention incorporating another embodiment of differential drive system, employing pinion gears, for discharging controlled volumes of fluid components from suitable fluid component sources such as reservoirs, the drive system being in a first position;
- Figure 6: is a right hand end view of the applicator as shown in Figure 5;
- Figure 7: is a view similar to Figure 5 with the drive mechanism in a second position;
- Figure 8: is a right hand end view of the applicator as shown in Figure 6;
- Figure 9: is a partial view of a preferred way of combining the volumes of fluid components discharged by the applicator shown in Figures 5-8 into an agent output;
- Figure 10: is a partial view of another way of combining the volumes of fluid components discharged by the applicator shown in Figures 5-8 into an agent output;
- Figure 11: is a plan view of another differential drive system, employing a pivoted lever;
- Figure 12: is a schematic plan view of another embodiment of an agent applicator according to the invention, which applicator employs a flow switching valve to selectively manage the outputs of two fluid component reservoirs;
- Figure 13: is a schematic plan view of a simple form of flow switching valve for use in the agent applicator shown in Figure 12;
- Figure 14: is a plan view of a modified form of selector for use in the agent applicator shown in Figure 12;
- Figure 15: is a side elevation of a proportioning valve for use in the agent applicator shown in Figure 12;
- Figure 16: is a plan view on the line 16-16 of Figure 15;
- Figure 17: is a plan view, partly in section, on the line 17-17 of Figure 15; and
- Figure 18: is a plan view, partly in section, on the line 18-18 of Figure 15.

### DETAILED DESCRIPTION OF THE INVENTION

The following more detailed description of the invention is intended to be read in the light of, or in context with, the preceding summary and background descriptions. Unless otherwise apparent, or stated, directional references, such as "up", "down", "left", "right", "front" and "rear", are intended to be relative to the orientation of a particular embodiment of the invention as shown in the first numbered view of that embodiment. Also, a given reference numeral indicates the same or a similar structure when it appears in different figures.

As disclosed above, the invention provides novel applicators for multi-component biologic and other fluids, which applicators and methods, inter alia, permit selective dispensing of a third fluid component, e.g., in the case of a sealant, a diluent or a therapeutic agent, in addition to the conventional sealant polymerization and activator agents. The invention also provides for the user to choose between activator agents of different strengths supplied from second and third fluid component sources to be output with a polymerization agent from a first source. In these and other ways, the invention provides the user novel options and capabilities not previously available. Such options include the ability to select a desired agent output from a range of agent outputs of different characters, or activities, including, for example, in the case of selant agents, sealants with various setting times and sealants with optional therapeutic properties provided by one or more auxiliary therapeutic agents. Further options can be provided by premixing the third component in the applicator with one or both of the conventional components.

The following detailed description will, by way of illustration, reference as examples of biologic fluid agents employable in the invention, fibrin sealants comprising a polymerizable fibrinogen agent and a thrombin activator agent, or equivalents thereof, and the surgical uses of the novel sealant application technology disclosed herein. It will, however, be understood by those skilled in the art, that the scope of the invention is not limited to fibrin or other biological sealants, and that synthetic and other sealants may be used for equivalent, or even for quite different, purposes from those described herein. It will also be understood that the applicators and methods described herein can be employed for applying, or dispensing, multi-component fluid products other than sealants which products benefit from being mixed at the point of application, or just prior to application. One example is that of products which are labile when their components are mixed. Another example is that of colored coating products, for example paints and enamels, including cosmetic nail enamels, which could be delivered from multiple components at the point of use, pursuant to the teachings herein, enabling the end user to control color by quantitatively or qualitatively controlling the delivery of components of the coating for mixing within a variable output applicator, or externally.

In order better to appreciate the scope of the invention and, in particular, the new capabilities it brings, inter alia, to the fibrin sealant art, it is helpful to understand certain characteristics of fibrin sealants. As described above, fibrin sealants generally comprise a fibrinogen component and a thrombin component. From the point of view of the user, e.g. a surgeon or other clinician, two parameters of importance are the setting time of the adhesive and its final hardness.

In general, the thrombin concentration determines the sealant setting time, or "setup". Preferred mixing ratios for fibrin sealants generally employ relatively high concentrations of thrombin, for hemostasis applications, where rapid control is desired, or relatively low concentrations, for slow-setting skin-graft or other bonding applications, where the surgeon may need time to manipulate the patient's tissue, or a prosthetic material or device, after the sealant is mixed and applied (or vice versa). For example, when a concentration of thrombin of at least about 100 IU/ml, preferably about 250 or more IU/ml, is combined with a fibrinogen concentration of at least about 25 mg/ml, preferably 60 mg/ml or more, (some commercial products can exceed 100 mg/ml), a fast-setting solid adhesive can be produced. Clotting times under 30 seconds, and as low as 10 seconds, are readily attainable.

Lower thrombin concentrations, below about 50 IU/ml, down to about 5 IU/ml, employing a similar fibrinogen concentration of about 60 mg/ml or more, yield a slow-setting sealant which may eventually becomes as hard or solid as the fast-setting adhesive, but may take several minutes to reach a comparable durometer value that a high concentration, 250 IU/ml thrombin formula yields in about 10 seconds.

On the other hand, it is the fibrinogen concentration that generally determines the final hardness of the adhesive. If the fibrinogen concentration is reduced below about 60 mg/ml, the gel becomes soft. Below 20 mg/ml, the resulting glue is quite runny. Accordingly, it is preferred to maintain a higher fibrinogen concentration above about 60 mg/ml for most applications, although lower fibrinogen concentrations may be desired for special purposes. An applicator, and application method, which provide the two options of a fast-setting, high thrombin adhesive and a slow setting, low thrombin adhesive, each employing a high fibrinogen concentration to yield a hard adhesive, and which enable a user quickly to switch between the fast- and the slow-setting adhesive, would be particularly useful. Such an applicator and method are provided by the present invention.

Turning to structural aspects of the preferred embodiments, the conventional fluid component sources can, as is known, comprise dischargeable reservoirs whose volumes can be varied, for example, syringes having movable plungers and, pursuant to the invention, the diluent, or third agent can also be sourced from a dischargeable reservoir, which may also, for example, be a plunger-equipped syringe.

In one embodiment of the invention, the plungers of all three syringes are coupled together by a coupling member in the form of a yoke so as to be advanced or withdrawn in unison, to vary the capacities of their respective reservoirs. With such an arrangement, the fluid from each and every reservoir is forcibly discharged to the sealant output, as the yoke advances, regardless of whether the user desires the full flow from each syringe to be included in the sealant output. This problem must be overcome if the user is to vary the output. To solve this problem the invention provides a flow switching device to switch the fluid component flow from at least one reservoir, and a waste chamber is preferably also provided to receive unwanted fluid from one or more reservoirs. Thus, for example, if the selector is set to dispense a high activity sealant, and the flow switching device provides a small proportion of diluent to the output, the waste chamber can receive from the flow switching device the balance of diluent discharged by travel of the respective plunger in the diluent syringe.

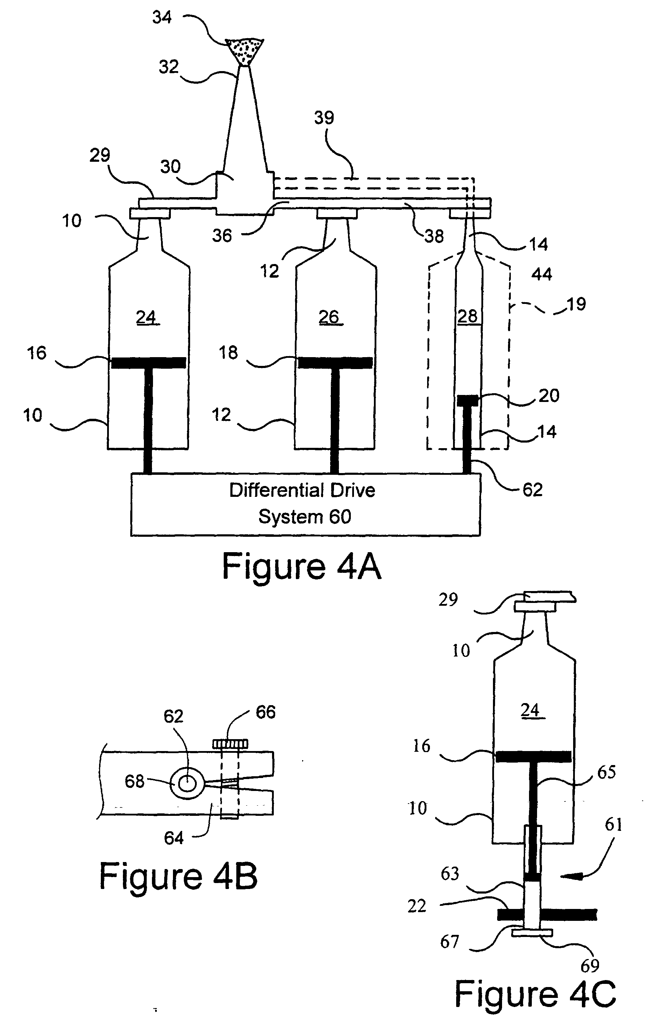

Pursuant to the invention, other, more sophisticated, drive systems, such as those described with reference to Figures 4A-C, 5-8 and 11, may provide for a differential drive as between one plunger and another whereby the operator can select from a number of proportionately different drive rates for example 1:1, 2:1, 3:1, or 1:2, and like so as to provide differential flow rates. Such drive mechanisms may, if desired be powered, for example electrically, possibly employing one or more stepper motors, or pneumatically or hydraulically.

Referring now to the embodiment of the invention illustrated in Figures 1-3, the sealant applicator shown comprises first, second and third syringes 10, 12 and 14, having plungers 16, 18 and 20 connected in harness by a yoke 22 to move in unison to vary the volumes of respective reservoirs 24, 26 and 28 in syringes 10, 12 and 14, whereby liquids in the reservoirs can be simultaneously discharged, with each plunger 16, 18 or 20 advancing at the same speed, simply by advancing yoke 22. Syringes 10, 12 and 14 thus provide suitable sources for up to three fluid components respectively stored in reservoirs 24, 26 and 28. Syringes 10 and 12, have similar sizes and volumes, as do their contained reservoirs 24 and 26. However, syringe 14 and its contained reservoir 28 have a substantially smaller transverse section, and therefore smaller volumes, than syringes 10, 12 and reservoirs 24, 26, for purposes to be explained below.

An applicator employing three syringes is disclosed in commonly owned Epstein copending application number 09/037,160. The syringes all discharge directly into a mixing chamber and there is no teaching of fluid component switching or of user-controlled variation of the sealant output, without refilling or reloading the syringes.

While the invention is not limited to any particular combination of fluid component sources, in the following illustrative embodiments, it may be assumed that a fluid fibrinogen agent of suitable concentration to provide a desired hardness in the resultant sealant is stored in reservoir 24, and that a fluid thrombin agent of suitably effective concentration, when delivered, is stored either in reservoir 26 or reservoir 28, as will be described in more detail below. The reservoir 26 or 28 in the third syringe 12 or 14 can store any one of a variety of helpful adjuvants or excipients that serve to give the user more choices, for example diluent, alternative activator components, therapeutics and so on.

If desired, syringe 14, while having a reduced internal volume as compared with syringes 10 and 12, can have a similar effective external volume 19, for example by having a thickened or cavity wall to be interchangeably received into the applicator with syringes 10 and 12. Alternatively, syringe 14 can be received into a bay and be equipped with fins or other lateral extensions to position it centrally in the bay with its nozzle or tip properly disposed, and its proximal face properly aligned with plunger 20, as though it were a full-sized syringe 10 or 12. Also, if desired, and in the interests of standardization and interchange ability, plungers 16 and 18 instead of filling the cross-section of syringes 10 and 12 can have a smaller cross-sectional size, like that of plunger 20. Reservoirs 24 and 26 could than have a movable closing plate (not shown) which is centrally recessed to receive the plunger 16 or 18.

This construction enables a plurality or range of syringe cartridges to be provided which dispense their fluid contents at different rates for a given rate of advance of yoke 22 in a manner providing modularity and convenience in the design of the applicator. For example thrombin agent may be made up to a standard concentration and filled into syringes, or syringe cartridges of different diameters and suitably labeled. The user then selects a larger diameter cartridge when desiring quicker sealant setup and a smaller diameter cartridge when desiring a slower setup. It will of course be understood that syringes 10, 12 and 14 can be constructed to be removable from the sealant applicator.

Syringe 10 communicates directly through a conduit 29 with an agent mixing chamber 30 from which a desired mixed sealant output 34 can be dispensed to a work surface via a dispensing cannula 32. Mixing chamber 30 preferably provides turbulent mixing, for example by direct, opposed impingement of the two sealant agent flows with one another. Preferably also, a clearing device or system is provided, for example a retrograde suction source, to clear clogs, debris, or unwanted residual sealant from the mixed sealant pathway comprised by mixing chamber 30 and dispensing cannula 32. Suitable turbulent mixing and clearing methods and devices are disclosed in application number 09/037,160.

Syringe 12, and its contained reservoir 26, communicate on one side of the output from reservoir 26, through conduit 36 with mixing chamber 30, and on the other side of the output, through conduit 38 with a two-way valve 40 which is a simple embodiment of flow switching valve. A further conduit 44 leads from two-way valve 40 to a waste chamber 46, which may be an expandable bladder, or other convenient variable volume, and may optionally be exhausted by suction. Syringe 40 and its reservoir 28 communicate with two-way valve 40.

Two-way valve 40 is manually operable, or can be driven by a manually operable two or three position, selector 48 through a coupling 42, to direct the output from reservoir 28 either to conduit 38 and thence to mixing chamber 30, or to waste chamber 46. The selector 48 shown in Figure 1 comprises a manually movable control such as a rotatable knob or lever or a slide control, accompanied by an indicator dial 50, which is mounted on the applicator in an externally accessible position and is movable to select the desired operating mode of two-way valve 40. As indicated by indicator dial 50, selector 48 can have positions for outputting from reservoir 26, from reservoir 28, and optionally, an "off' position. Other mechanical or even electrical or electronic devices enabling a user to control two-way valve 40 will be apparent to those skilled in the art, including for example, a movable lever, push-button, depressable key, slide or rocker.

Clearly, too, the hydraulically functional components of two-way valve 40 may take many forms. One possible construction is indicated schematically in Figure 2 wherein two-way valve 40 comprises a rotatable valve body 52 having a fluid passage 54 which turns through a right angle. In the position shown in full lines in Figure 2 fluid passage 54 puts reservoir 28 in syringe 14 in communication with conduit 38 leading to mixing chamber 30. When valve body 52 is rotated through 90 degrees, in the direction of arrow 56, fluid passage 54 assumes the position shown in broken lines where syringe 28 communicates with conduit 44 and thence with waste chamber 46.

Preferably, yoke 22 is a rigid, possibly integral, or even unitary construction so that plungers 16, 18 and 20 move in unison, advancing at the same speed, when a drive impulse is applied to yoke 22. However, a useful alternative construction is for yoke 22 to have a joint 58 closable by a clasp 59 to enable plunger 28 in syringe 14 to be disconnected from yoke 22. Thus, when the fluid component in reservoir 28 is not required in sealant output 34, clasp 59 can be opened so that no fluid is dispelled from reservoir 28 as yoke 22 is advanced. When the reservoir 28 component is required, clasp 59 is closed and plunger 20 moves in unison with plungers 16 and 18. Closable joint 58 thus provides a simple alternative to two-way valve 40 and waste chamber 46. Other ways of separably connecting plunger 20 to yoke 22 will be readily apparent to those skilled in the art..

In the modification shown in Figure 3, mixing chamber 30 and cannula 32 are replaced by separate dispensing cannulas 70 and 72, which may equally well be conduits in a single nozzle. Cannula 70 outputs the single agent from reservoir 24, e.g. a fibrinogen agent, while cannula 72 outputs the agent from reservoir 26, or a combination of the agents in reservoirs 24 and 26, as determined by selector 48. Cannulas 70 and 72 dispense side-by-side streams or drops of sealant for mixing on the work surface, as is known to those skilled in the art, and provide a general alternative to the use of internal mixing chamber 30 and a single mixed sealant dispensing cannula 32 for the applicators described herein. The sealant produced will lack the quality advantages of internal impingement mixing, but otherwise is expected to have the benefits of the invention described herein.

As plunger 16 is driven along syringe 10, by advancing yoke 22, under user control, upwardly in the sense of Figure 1, any fluids in reservoir 24 are discharged, or dispelled, into conduit 29 at a rate which is predetermined and fixed in relation to the rate of advance of plunger 16. Absent obstructions, the rate of discharge will depend primarily upon the geometry of syringe 24. Conduit 29 directs the complete output of reservoir 24 to mixing chamber 30. Similarly, plunger 18 is advanced by yoke 22 and discharges the fluid contents of reservoir 26 directly into conduit 36 at a rate determined by the rate of advance of yoke 22. All the contents of reservoir 26 are accordingly supplied to mixing chamber 30 via conduit 36. In contrast, the destination of the contents of reservoir 28, which is also discharged at the same time and the same speed as reservoirs 24 and 26, can be varied as between conduit 38 and mixing chamber 30 whence the contents will become a component of sealant output 34, on the one hand, or be deposited in waste chamber 46, on the other hand. The destination depends upon the position of valve body 52 in two-way valve 40, which is determined by manual selector 48.

The embodiment of Figures 1-3 provides a simple and practical way of optionally adding a third fluid component to the sealant output 34. Many possible uses of such an applicator will be apparent to those skilled in the art from the teaching herein. For example, second syringe 12 can contain a low-concentration thrombin agent, while third syringe 14 with its relatively small volume can contain a more highly concentrated, higher activity thrombin as the third agent. With two-way valve 40 in the broken line position of Figure 2, the third agent is discharged to waste chamber 46, yielding a slow glue or sealant. With two-way valve 40 in the full line position the third agent is combined with the second, changing the volume by a minor amount, and yielding a much faster glue with a higher thrombin concentration. Alternatively, third syringe 14 can contain a therapeutic agent, or equivalent, which is either used or not used depending upon the user-controlled setting of selector 48.

Thus, reservoirs 26 and 28 can contain any desired combination of fluid components for incorporation in a mixed sealant output 34 and which can be satisfactorily fed through conduits 36 and 38. Because the mixed fluids pathway constituted by conduit 36, when both reservoirs 26 and 28 are selected, is not subject to clearing, fibrinogen and thrombin, or another gellable combination of polymerization and activator agent, should not both be included in reservoirs 26 and 28. Nor need these two reservoirs necessarily provide the source for thrombin. For example, reservoir 24 could contain a desired thrombin concentration, and reservoir 26 could contain a fibrinogen agent, while reservoir 28 provided an auxiliary agent, for example an agent beneficially mixed with the fibrinogen component such as a source of a reinforcing material, for example elastin or collagen. If desired, this auxiliary material can be provided in varying relative concentrations, employing one or other of the teachings hereinbelow.

Where the surgeon encounters situations requiring two different types of sealant which can be met by a combination of the fluid component in reservoir 24 with that in reservoir 26 or in the alternative with the component in reservoir 28, the surgeon can perform a method of sealant application comprising the following steps:
a) movement, if necessary, of selector 48 (or verifying its setting) to select a first desired sealant output;
b) actuation of the applicator to dispense the first sealant output to the work surface or an appropriate portion thereof;
c) movement of selector 48 to a second position to select a second desired sealant output; and
d) further actuation of the applicator to apply the second sealant output to the work surface or to an appropriate portion thereof.

The application of the second sealant output can be effected without dismantling the applicator or otherwise manually replacing, exchanging or re-connecting a fluid component source simply by moving selector 48. Preferably, as described in the above-referenced copending applications, where internal mixing of the fluid components within the applicator is employed, the mixed component pathway comprised for example by mixing chamber 30 and cannula 32, or the like, is cleared of residual materials between applications of the first and second sealant outputs, for example by applying retrograde suction. Still more preferably, it is desirable for the user to effect such clearing within a time interval after sealant dispensing stops which is less than the setup time of the sealant, to remove residual fluid sealant in mixing chamber 30 and cannula 32 before it gels.

The applicator provides the surgeon or other operator a flexible range of options as will be apparent to those skilled in the art. For example, to seal a flap of tissue employing an applicator as illustrated in Figures 1-3, charged with fibrinogen in syringe 10, a low thrombin concentration in syringe 12 and a high thrombin concentration in syringe 14, a surgeon may first select a high thrombin setting of selector 48 to provide a fast-acting sealant, using high thrombin concentration from syringe 14. The surgeon then applies a spot pattern of this fast-acting sealant to the flap to tack it in place. Prior to changing the setting of selector 48, if desired, the operator can clear mixing chamber 30 and dispensing cannula 32 of any clogging material or residual fast-acting sealant, for example by retrograde suction, or other means, if available. Preferably, the clearing action is initiated promptly after cessation of sealant flow to remove residual mixed fluid components before setup. Selector 48 is then moved to a setting discharging a low concentration thrombin from syringe 12 to provide a slower sealant output 34, which is now dispensed along the edge of the tissue flap to seal it in place. Again, the applicator is cleared of clogs or residuals before reuse.

This, first embodiment of the invention, as illustrated in Figures 1-3, provides the user with a simple applicator and method yielding a variable sealant output which can readily be selected, or determined, by the user. Once selected, a constant character sealant output is provided, until the selection is changed (or one or more fluids becomes exhausted).

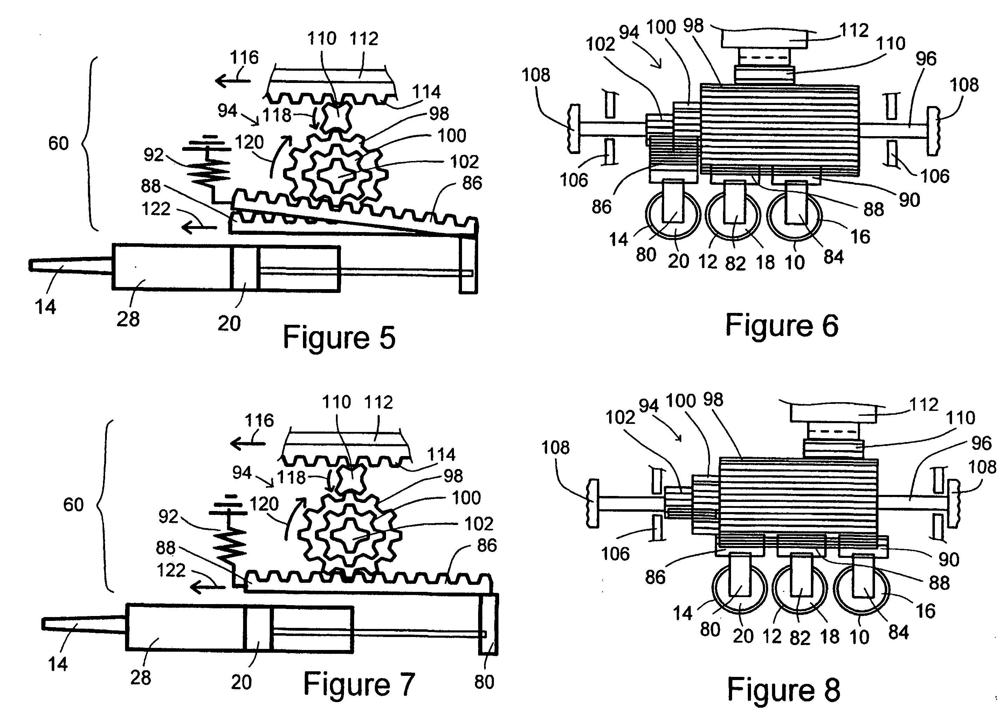

The range of possible sealant outputs is first matched to the user's needs by suitably selecting three fluid components and charging them into reservoirs 24, 26 and 28. To vary the character of the sealant output 34 delivered from reservoirs 24, 26 and 28 to cannula 32 (or cannulas 70 and 72), it is necessary for the sealant delivery system, which supplies a desired combination of fluids from reservoirs 24, 26 and 28, to include a switching device responsive to the user's selections to effect a variation in fluid flow of one of the sealant sources to cannula 32, with respect to the flow from one or both of the other sources. In the embodiment of Figures 1-3, the switching device comprises a simple two-way valve 40 which switches the fluid flow output from reservoir 28 either to sealant output 34, via mixing chamber 30 and cannula 32, or to waste chamber 46. Alternatively, the switching device can switch the drive impulse applied to at least one of the plungers, for example, the drive impulse applied to plunger 20 to drive it at a different speed with respect to another plunger, 16 or 18. One such apparatus, employing a switchable differential drive system, utilizing pinion gears, is illustrated in detail in Figures 5-8.

Some of the benefits obtainable by employing a differential drive system can be understood from the system illustrated schematically in Figure 4A, where a differential drive system 60 is shown replacing yoke 22 of Figure 1. While the system illustrated and described in connection with Figures 4-9 relate to the application of differential drive system 60 to multiple plungers 16-20 to discharge fluid components from reservoirs 24-28, it will be understood that differential drive system 60 can also be employed, with advantage, with other structures designed to discharge multiple fluids from storage, for example levers acting on bladders, as referenced elsewhere herein, or could be adapted to control valving for an effectively continuous supply of one or more of the fluid components, or to control valving for the supply of one or more of the fluid components from a source or sources external to the applicator.

Referring to Figure 4, as illustrated, differential drive system 60 controls the advance, and optionally the retraction (for example for filling purposes) of each of plungers 16, 18 and 20. At least one plunger, plunger20 in reservoir 28 in the illustration, is driven independently and at a different rate from one or both of the other plungers. In the particular case shown plunger 20 has advanced a smaller fraction, perhaps 50 percent, of the distance traveled by plungers 16 and 18. Consequently, if reservoir 20 had the same cross-sectional area as reservoirs 24 and 26, about 50 percent as much fluid would be discharged from reservoir 28 as from each of reservoirs 24 and 26. In the particular case shown, reservoir 28 has a reduced cross-sectional area as compared with reservoirs 24, 26 and the proportion of fluid discharged is correspondingly reduced.

In the system of Figure 4A, there is no switching device, such as valve 40, between reservoirs 24-28 and sealant output 34. Instead, reservoirs 24 and 26 feed directly to mixing chamber 30 via conduits 29 and 36, respectively, while reservoir 28 feeds to mixing chamber 30 via conduits 36 and 38, without interruption to the flow path, except as is provided by fluid from-reservoir 26 in conduit 36, and possibly also in conduit 38. If desired, reservoir 28 could feed directly to mixing chamber 30, via conduit 39, shown in broken lines. Accordingly, to provide a variable sealant output, it is desirable for differential drive system 60 to include a switching device readily activatable by an external control such as manual selector 48, to change the speed of advance of one of plungers 16-20, or to disconnect a plunger from, or re-connect a plunger to, the applied drive. Although differential drive system 60 is described in the context of an array of three syringes 10-14, it will be clear that more than three syringes could be employed, if desired.

Another useful system (not shown) employs two syringes and the speed of travel of one can be varied in relation to the other by means of differential drive system 60. In this case one syringe could, for example, dispense fibrinogen, at a desired rate, while the other syringe dispenses thrombin at rates subject to selection by the user, to provide sealants with different setups.

It will be understood by those skilled in the art that differential drive system 60 can be embodied in a wide variety of forms to enable a user quickly and easily to dispense one fluid component at a different rate from another, and preferably also to change that rate, in a manner that will not significantly interrupt the workflow of say, a surgeon. Possible embodiments include gear, lever, line or belt drive and limited slip drive mechanical embodiments as well as hydraulic, pneumatic and electrical or electronic embodiments.

Thus, in one simple system, illustrated in Figure 4B, differential drive system 60 comprises a yoke similar to yoke 22 with the addition of a limited slip coupling to plunger rod 62 of plunger 20, generating lost motion in plunger rod 62 as the yoke is advanced, so that plunger 20 travels a shorter distance in a given time than does plunger16 or plunger 18.

The limited slip coupling shown in the drawing, by way of example, comprises a split collar 64 fixed to, or in this case, integral with yoke 22, and sliding on plunger rod 62, which split collar 64 is tightenable with a screw control 66, which could equally well be a lever. Screw control 66 enables the degree of friction between plunger rod 62 and split collar 64 to be varied to provide a desired degree of slippage to control the speed of advance of plunger 20. Screw control 66 can bear visual markings to indicate the degree of friction and the anticipated correlation with rate of discharge of reservoir 28. Preferably, split collar 66 is relatively rigid and a more resilient insert 68 is provided, within split collar 66, to provide a desired range of frictional force.

Since the manually applied drive forces are relatively modest, plunger rod 62 can be highly polished to provide adequate slippage, and insert 68 can be configured to engage plunger rod 62 over a relatively narrow zone to reduce friction, for example by having a triangular cross section with an apex engaging the surface of plunger rod 62. The triangular section of insert 68 can help provide a suitably graded reduction in slippage as tightening of screw 66 increases the surface area in contact with plunger rod 62, as well as the applied force. The material of insert 68 is selected for its frictional characteristics, and depending upon the frictional force required in a particular embodiment may range from a rubbery, high-friction elastomer, to a slippery low friction, material such as a polyfluorinatedalkylene. Screw 66 acts as a switching device enabling different rates of travel of plunger 20 to be selected by suitable adjustment of the screw.

In the system shown in Figure 4C, differential drive system 60 comprises another embodiment of modified yoke wherein syringe 10 is provided with a variable drive relationship to syringes 12 and 14 by employing a pneumatic damper 61 as a telescoping plunger rod for plunger 16. Damper 61 has a cylinder 63 connected to yoke 22, while a piston 65 is a close sliding fit within cylinder 63 and is preferably provided with adequate seals for airtight engagement with cylinder 63. Syringes 12 and 14 are driven in unison by yoke 22 at the same speed as each other, as described above. At yoke 22, pneumatic damper 61 is provided with a bleed valve 67 controlled by a rotatable, and preferably calibrated, screw 69. The user can set screw 69 to determine the air bleed through valve 67 and thus control the travel of piston 65 in cylinder 63, thereby controlling the degree of lost motion in the advance of plunger 16 relative to plungers 18 and 20.

Once selected, a constant character sealant output is provided, until the selection is changed (or one or more fluids becomes exhausted). While the lost motion drives of the embodiments of Figures 4B and 4C can provide a reasonably constant output, it will be appreciated that a positively acting differential drive, not dependent upon lost motion, may provide a more consistently constant output.

In the embodiment shown in Figures 5-8, one form of differential drive system 60 uses a single gear to drive two of the three syringes and a selectable gear to drive the third syringe, enabling the third agent to be dispensed in selectable controlled volumes for unit travel of the respective plunger. Referring to Figures 5 and 6 syringes 10, 12 and 14 are each of similar construction and similar internal volumes. The relative positions of syringes 10 and 14 are switched left to right, as compared with their positions in other embodiments, in order to continue to reference syringe 14 as a source of variability. The outputs of syringes 10-14 are connected directly to a mixing chamber 30 or cannulas 70, 72 or 70-74, as shown in Figures 3, 4, 9 or 10, via suitable conduits, as shown, but without need for a switching valve such as valve 40 to obtain a variable output which is instead obtained by operation of differential drive system 60.

The respective plungers 16, 18 and 20 each have a plunger rod terminating in a drive member 80, 82 or 84, respectively. Drive members 80-84 are coupled to respective toothed racks 86, 88 and 90 extending longitudinally above syringes 14, 12 and 10. Rack 86 for syringe 14 is pivotally attached to drive member 80 at its right hand end and its left-hand end is biased upwardly by a tension spring 92. A compound gear 94 is rotatably mounted on a shaft 96 (not shown in Figures 5 and 7). Compound gear 94 comprises a primary pinion gear 98 which engages and drives racks 88 and 90, and optionally rack 86, and two successively smaller secondary pinion gears 100 and 102, which can selectively engage rack 86. Secondary gears 100 and 102 have a width comparable with or somewhat less than that of toothed rack 86 while it is preferred that primary pinion gear 98 has a width sufficient to engage both racks 88 and 90, and more preferred that the width be sufficient to engage all three racks 86-90 simultaneously, as shown in Figure 8.

Referring to Figure 6, compound gear 94 is laterally movable with shaft 96, so that rack 86 driving plunger 16 in syringe 14 can be selectively engaged with any one of the three pinion gears 98-102, providing three different drive ratios, one of which is the same as the drive ratio for racks 88 and 90. The ends of shaft 96 are journalled into walls 106 of a housing for the applicator and terminate externally in finger grips 108 (For clarity, walls 106 and finger grips 108 are not shown in Figures 5 and 7). Shaft 96 extends beyond walls 106 by an amount which slightly exceeds the combined width of pinion gears 100 and 102, so that shaft 96 can be manually moved laterally back and forth to position a desired pinion of compound gear 94 in engagement with toothed rack 86. Finger grips 108 preferably bear an external visual, graphical, or colored, or both graphical and colored, indication of the engagement position of shaft 96.

A suitable shift lever or latch, or other effective device (not shown), can be provided to depress the left-hand end of toothed rack 86 to accommodate the larger pinion gears 100 or 98 as they are moved into engagement with toothed rack 86. The shift lever can be operated by a separate manual control, but is preferably coupled with shaft 96, for example, by a cam or the like, so that only one manipulation is required to shift gears. For example, a push button could be incorporated in one or the other of finger grips 108 and could be connected to depress toothed rack 86, or to release spring 92 allowing rack 86 to drop. Preferably, the push button is resiliently biased by spring 92, or by other means. Such a selector lever can be mounted to be externally accessible above the applicator and to have a compound movement whereby it is depressed downwardly to depress rack 86 and then swung or shifted laterally to move compound gear 94.

Preferably also, such a shift lever, or push button, is releasably lockable for example by engagement of a detent in a recess, into a position which holds pivoted rack 86 in its downward or depressed position disengaged from gears 100 or 102, while gear 98 is engaged only with racks 88 and 90. This feature provides the user the option of dispensing nothing from reservoir 24 and, in this sense, is thus a functional equivalent of two-way valve 40 in the embodiment of Figures 1-3, enabling syringe 10 to be turned off.

Various structures can be employed to translate a manually applied impulse, or impulses, from the user to rotate compound gear 94 on shaft 96. Preferably, a mechanism that can increment successive impulses is employed, for example a trigger-actuated rack-and-pawl mechanism such as that described in copending application number 08/946,364. One possible way of integrating the differential drive mechanism of the present invention with that trigger-actuated mechanism is suggested schematically in Figures 5 and 6. For this purpose an idler pinion 110 is located above primary pinion gear 98, in engagement therewith, and is rotated by a further toothed rack 112. Toothed rack 112 is similar to the pawl-driven toothed rack referenced 50 in application number 08/946,364, with the addition of teeth 114 on the underside of the rack. Thus toothed rack 112 preferably has a length sufficient to permit full-length travel of a plunger in a syringe, and is advanced by successive engagement of a pawl with rows of teeth on an upper face of the rack (not shown).

As rack 112 is advanced forwardly, in the direction of arrow 116, it turns idler gear 110 in the counter clockwise direction of arrow 118, as viewed in Figure 5. Idler gear 110 rotates primary pinion 98 in the clockwise direction of arrow 120, driving racks 86-90 forward in the direction of arrow 122. In the position shown in Figures 5 and 6, primary pinion gear 98 drives racks 88 and 90 while pivoted rack 86 is driven by secondary pinion gear 100, and thus advances at a different, faster speed than racks 88 and 90, pinion gear 100 having fewer teeth than pinion gear 98. In the position shown in Figures 7-8, the assembly of shaft 96 and compound gear 94 has been moved to the left, pivoted rack 96 having been depressed, so that now primary gear 98 engages all three racks 86-92. Plungers 16,18 and 20 accordingly move in unison, at the same speeds, as though pinion gear 98, racks 86-92 and drive members 80-84 comprised a fixed yoke, such as yoke 22 (Figures 1-3).

The ratio of the speed of advance of plunger 16 to that of plungers 18 and 20 is the ratio of the number of teeth on gear 98 to the number of teeth on gear 100, or 102, if the latter were the secondary pinion gear engaged. The ratio can of course also be varied by varying the pitch of the teeth on rack 86 as compared with that of rack 88 or 90. Clearly racks 88 and 90 must have the same pitch in order to be driven by a single gear, gear 98. The teeth on racks 112 and 122 should have the same pitch so that they advance at the same speed and to avoid gearing difficulties.

In use, syringes 10, 12 and 14 are charged with fluid components selected according to the available relative drive speed or speeds for a particular syringe. Thus, for example, syringes 10 and 12 may accommodate fibrinogen and thrombin components, respectively, while syringe 14 receives a treatment agent, for example an antibiotic, to be dispensed at any one of three selected rates according to which of the three pinion gears 98, 100 and 102 is engaged by rack 86. The surgeon, or other user, checks the visual indication provided by finger tabs 108, or provided elsewhere, to ensure that differential drive system 60 is set to a desired combination of ratios, such as shown in Figure 6 where plunger 20 will advance relatively more slowly in syringe 14, than will plungers 16 and 18 in syringes 10 and 12. With the desired drive ratios properly selected, the user manually actuates the applicator, for example by depressing trigger 22 (see application number 08/946,364) to dispense a first sealant output 34 comprising a mixture of equal amounts of components from syringes 10 and 14, with a lesser amount of component from syringe 14.

For example, the surgeon may apply a fibrin sealant along with a relatively lower concentration of antibiotic to more sensitive inner wound tissues. Manual movement of an external actuator such as trigger 22 (see application number 08/946,364), repeated if necessary, advances toothed rack 112 in the direction of arrow 116, rotating idler gear 110 and compound gear 94 in the directions of arrows 118 and 120, respectively (Figure 5). Gear 98 advances racks 88 and 90, and their connected plungers 16 and 18, at full speed, being the same speed as the speed of rack 112, dispensing fluid components from reservoirs 24 and 26 (in syringes 10 and 12) at one rate, while gear 100 advances rack 86 and plunger 20 at a fraction of full speed to dispense the fluid component from reservoir 28 at a proportionally lower rate according to the ratio of the number of gear teeth on gear 100 to the number on gear 98.

To dispense an agent output 34 of different composition, for example with a higher concentration of antibiotic for treating the outer areas of a wound, the surgeon selects a new operating position of differential drive system 60, providing a different combination of plunger advance speeds, by gripping finger tabs 108 and moving compound gear 94 and shaft 106 to the left from the position shown in Figure 6 to that shown in Figure 8. Rack 86 is pivoted downwardly to accommodate leftward movement of gear 98 in any convenient manner, for example by a cammed lever, as described above. Optionally, the mixed fluids pathway can be cleared of debris or residuals, as taught in the copending applications, before or after switching the differential drive system 60. The user then actuates the applicator to dispense the new combination of fluid components as sealant output 34 with plunger 20 traveling at the same rate as plungers 16 and 18, providing equal proportions of all three fluid components in the sealant output 34.

Use of gearing, as opposed to a limited slip or other less positive drive mechanism, ensures that the factory designed speed ratios are maintained. Different ratios and sets of ratios can be accommodated by providing for interchangeability of pinions 98-102 on shaft 96 and, if desired, providing the user with a full set of pinions of different widths and diameters from which to select combinations of pinions to provide desired combinations of drive ratios, thus permitting the applicator to be set up for a variety of different sealant output combinations.

Within the practical engineering limits of any particular embodiment, pinion gears 100 and 102 can be selected to provide any desired drive ratios, giving plunger 20 the choice of two slower speeds than that of plungers 16 and 18. Thus, exemplary pairs of speed ratios for plungers 16 and 18 with respect to plunger 20 are 2: 1 and 3:1, 4:1 and 6:1, 5:1 and 3:1, 5:4 and 3:4 and so on. Other arrangements of compound gear 64 can provide for the larger gear 98 to drive only one plunger 16, while two or more smaller gears, such as gears 100 and 102, can be selectively applied each to drive one or both of plungers 18 and 20. It will be understood that the plunger, or plungers, that is, or are, to be driven faster should be driven by the larger gear 98 and that different combinations of the dispensing speeds of individual fluid components can be obtained not only by movement of compound gear 94 to have a different pinion engage a different rack, which can possibly be effected during the course of a surgical procedure, but also by changing which sealant component is contained in which syringe 10, 12 or 14.

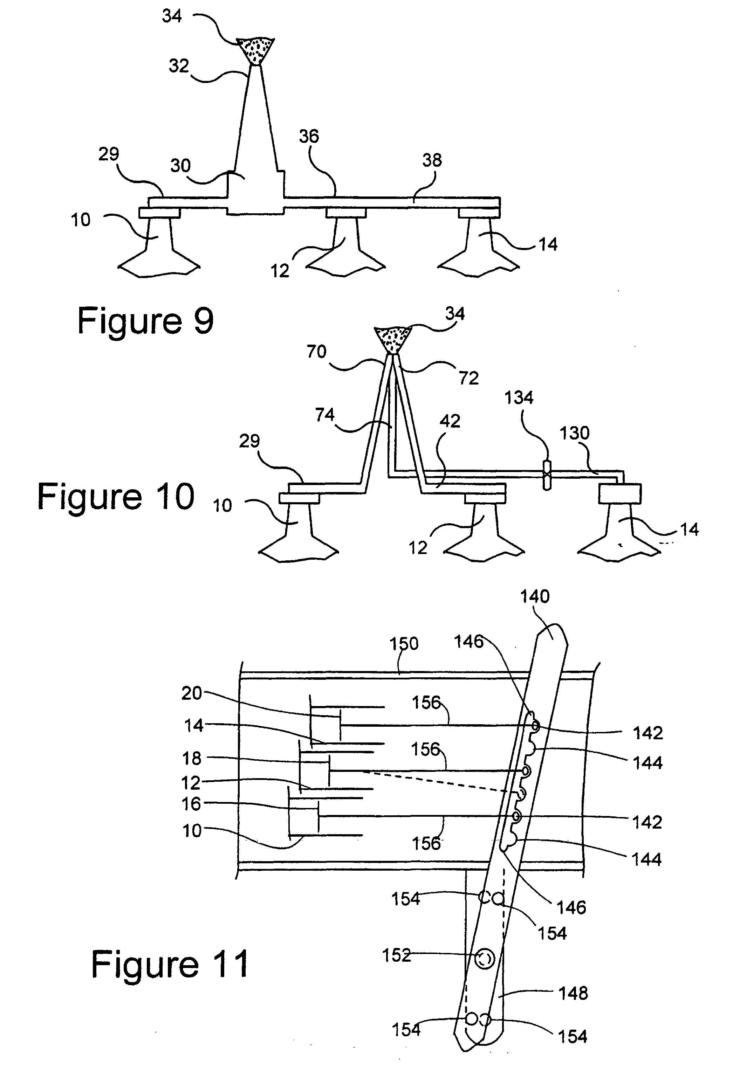

Figure 9 illustrates one preferred way of combining the outputs from syringes 10-14 as they are disposed in the embodiment of Figures 5-8, in a manner providing internal mixing of fluid components. Syringe 14, being the syringe subject to a differential drive at a speed that can be varied to be different from that of syringes 10 and 12, discharges its output directly through conduit 38 to be mixed in conduit 36 with the output from syringe 12. This combined output is then further mixed in mixing chamber 38 with the output from syringe 10. If preferred, the output from the syringe subject to a differential drive could be supplied directly to mixing chamber 30, e.g. from syringe 10 through conduit 29, so long as two participants in a gelling reaction are not both supplied to mixing chamber 36 through a common conduit, such as 29.

Figure 10 illustrates an alternative construction for dispensing three fluid components from individual syringes 10, 12 and 14 for external mixing. Each syringe 10, 12 or 14 is discharged through a respective conduit 29, 42 or 130 to a respective dispensing cannula 70, 72 or 74. The three cannulas 70-74 meet at a common point to provide an agent output 34 containing ingredients from all three cannulas which may or may not require further mixing to provide an effective sealant.

When used with a suitable differential drive system 60, as described herein, this arrangement can also provide a variable sealant output having a variable agent concentration or composition. Optionally, a shut-off valve 134 can be provided in conduit 130 enabling the user to determine whether the fluid component contained in syringe 10 should be present in sealant output 34. Clearly, shut-off valve 134 could additionally, or alternatively, be present in conduit 29 or conduit 42. To avoid difficulties, it is preferred that the syringe, if any, discharging to shut-off valve 134 be driven by a lost motion mechanism such as is shown in Figure 4B or Figure 4C, so that back pressure from closure of shut-off valve 134 simply forces plunger rod 62 or 65 to move relative to yoke 22, rather than with respect to syringe 10, 12 or 14.

An equivalent arrangement can be used with applicators employing four or more syringes discharging into two three or four cannulas with mixing of non-gellable combinations of components, if desired.

As with the Figure 3 embodiment, individual discharge of the fluid components in the manner shown in Figure 10, lacks the advantages obtained by mixing the components internally within the applicator, before the components exit the applicator, which advantages are discussed more fully in the copending applications.

Figure 11 illustrates a pivoted lever embodiment of differential drive system 60 wherein a lever 140 functions as a yoke driving plungers 16, 18 and 20 into syringes 10,12 and 14 in unison, but at different speeds. Plungers 16, 18 and 20 each have beaded ends 142 accommodated in grooves 144 formed in an elongated slot 146 in lever 140. Lever 140 is pivotally mounted on an outrigger 148 extending from an applicator body 150, which houses syringes 10, 12 and 14, and by means of a peg 152 inserted into registering holes 154 in any one of multiple positions, three shown. Lever 140 projects through slots (not shown) in the sides of applicator body 150. The user selects a suitable pair of pivot holes 154 to provide a desired set of drive ratios. The ratios are determined by simple geometric considerations such as the spacing beaded ends 142 and peg 152 along lever 140, as will be readily apparent to those skilled in the art.

In an alternative arrangement (not shown) a first and second of plunger rods 156 are coupled together by a regular, non-pivoted yoke to be advanced in unison at the same speed as one another. The yoke can carry a cam surface, or other means, provided for example, by a rounded peg, bearing against the proximal surface of lever140 to pivot lever 140 forwardly and move the third plunger, coupled to lever 140, at a speed different from that of the first two plungers.

By providing multiple grooves 142 from which to select a groove to receive beaded end 142 of a given plunger 16, 18 or 20, the individual plunger's drive ratio is also made selectable. To accommodate the arcuate movement of lever 140 about peg 152, plungers 16, 18 and 20 can have flexible shafts, or beaded ends 142 can ride in slots (not shown) in lever 140, which preferably have an arcuate shape centered on peg 152. The drive ratios of the three plungers 16-20, taken as a group can be switched by moving peg 152 to a different pair of holes 154 while an individual drive ratio can be varied by switching beaded end 146 to a different groove 144.

As the lever140 is advanced manually, or by a suitable drive mechanism, to pivot about peg 152, plungers 16, 18 and 20 are driven along syringes 10, 12 and 14. Plunger 20 travels faster than plunger 18 while plunger 16 travels slower than plunger 18, the particular speeds being determined by the relative spacing from peg 152. The pivoted lever differential drive mechanism shown in Figure 11 can be readily adapted to engage and retract the plungers, as well as to advance them, if desired.

Many different combinations of fluid components can be deployed in the three syringes 10-14 to take advantage of the novel differential drive capability of the invention. Some of the possibilities will now be described and others will be apparent to those skilled in the art. In the following description, it will be assumed that syringe 14 is subject to a selectable differential drive enabling it to be advanced at a speed different from that of syringes 10 and 12, and that the outputs from the several syringes 10-14 are combined internally, for example as shown in Figures 1, 4, 9 and 12, or externally, as shown in Figures 3 or 10.

As has already been discussed, the fluid components can include, besides the active participants in a gelling reaction, namely a gelling, or polymerizable agent and an activator or catalyst for the reaction, auxiliary agents, supplemental sealant agents, diluents, therapeutic agents and other such useful agents that can be conveniently delivered by the sealant output. Of particular interest is the ability to vary the concentration of one or more agents, especially the concentration of thrombin. Also of interest is the ability to add a third agent, especially a therapeutic agent such as an antibiotic, or one or more cellular growth factors or other components that can promote wound healing or protect the healing wound from infection.

In the case of fibrinogen sealants used for surgical purposes, several useful exemplary combinations of fluid components employ a fibrinogen agent in syringe 10. For example, syringe 12 can contain calcium chloride, or another suitable thrombin agent diluent, and the third syringe, syringe 14 can contain thrombin to be added to the diluent from syringe 12 in variable concentrations, according to the setting of the differential drive system, and provide an agent output with a variable setup. Using the geared differential drive system 60 shown in Figures 5-8, three different sealant setting times may be selected by switching between gears 98, 100 and 102 for example, by manually laterally shifting compound gear 94 on shaft 96 using finger grips 108.

In another example, syringe 12 can contain a low concentration thrombin agent, while syringe 14 contains a high concentration thrombin agent which is mixed with the low concentration thrombin agent from syringe 12, in conduit 36, (unless external mixing is employed, as described with reference to Figure 10), to provide an agent output with a variable setup or setting time. Four different sealant setups may be selected using the geared differential drive system 60 shown in Figures 5-8, if it includes a no-drive option in which rack 86 is held clear of engagement with compound gear 94. The slowest setup, as determined by the thrombin concentration in the sealant output 34 is obtained with rack 86 disengaged so that no high concentration thrombin agent is discharged from syringe 14 to the sealant output 34, and progressively quicker setups are obtained by driving rack 86 with pinions 102, 100 and 98, in that sequence.

In a further useful example, syringe 12 contains thrombin, and variable drive syringe 14 contains a therapeutic agent which can be dispensed in various concentrations selected by the user, by suitably positioning compound gear 94.

Other embodiments of differential drive system 60 will be known or apparent to those skilled in the art. For example, one or more hydraulically driven plungers or systems could be used in one or more syringes 10-14. Analogously to an automotive braking system, the applicator drive system could comprise a master hydraulic cylinder pressurized by the user by operation of a manual actuator and driving one or more independently acting slave cylinders to discharge one or more syringes 10-14. A manually operated switching device could switch one or more of the slave cylinders into and out of operation, or preferably could actuate one or more valves to vary the pressure applied to fluid in a respective syringe reservoir 24-28 by one or more of the slave cylinders thus providing a differential drive system. If desired, an equivalent electrically powered drive could be provided by employing stepper motors, linear motors, or the like, along with suitable user-operated electrical or electronic switching controls.

Where the fluid components are provided from a pressurized source or sources, possibly external to the applicator, through individual fluid conduits, differential drive system 60 can act to pressurize the sources and one or more manually selectable valves can be provided to vary the flow rate of, or switch on and off, or divert, the fluid output from at least one of the sources to the sealant output 34.

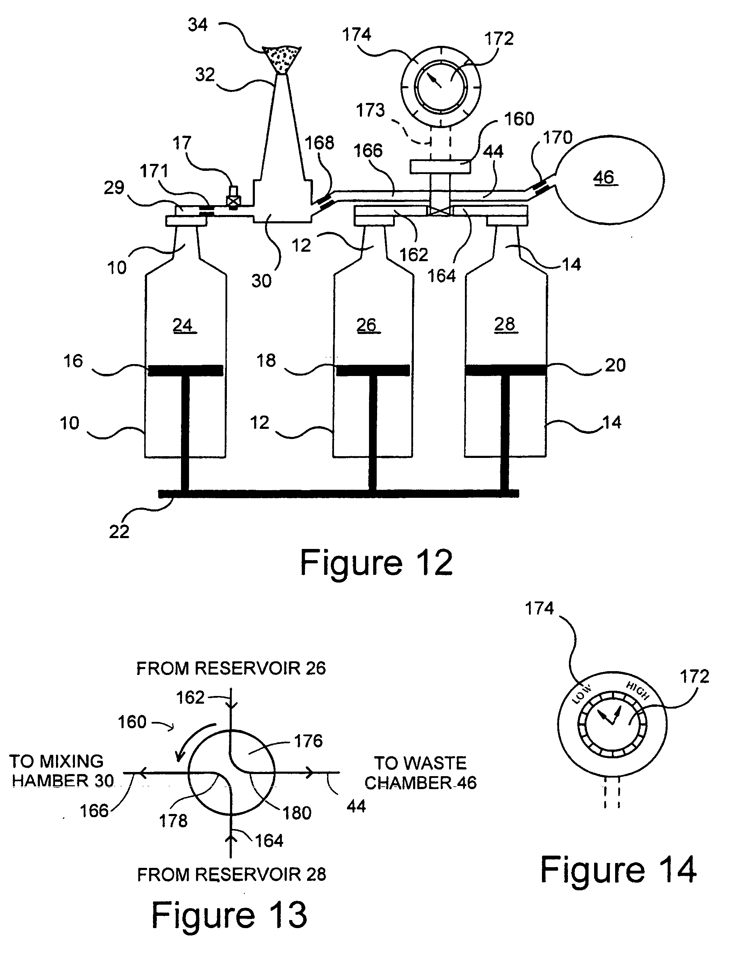

Like the embodiment of Figure 1, in the embodiment of applicator shown in Figure 12 three syringes 10, 12 and 14 have reservoirs 24, 26 and 28 dischargeable by plungers 16, 18 and 20 which are driven in unison, each at the same speed, by a yoke 22. If desired, yoke 22 could be broken by a joint 58 closable with a clasp 59, although same is not shown. Syringe 10 discharges through a conduit 29 to a mixing chamber 30, a cannula 32 and to sealant output 34.

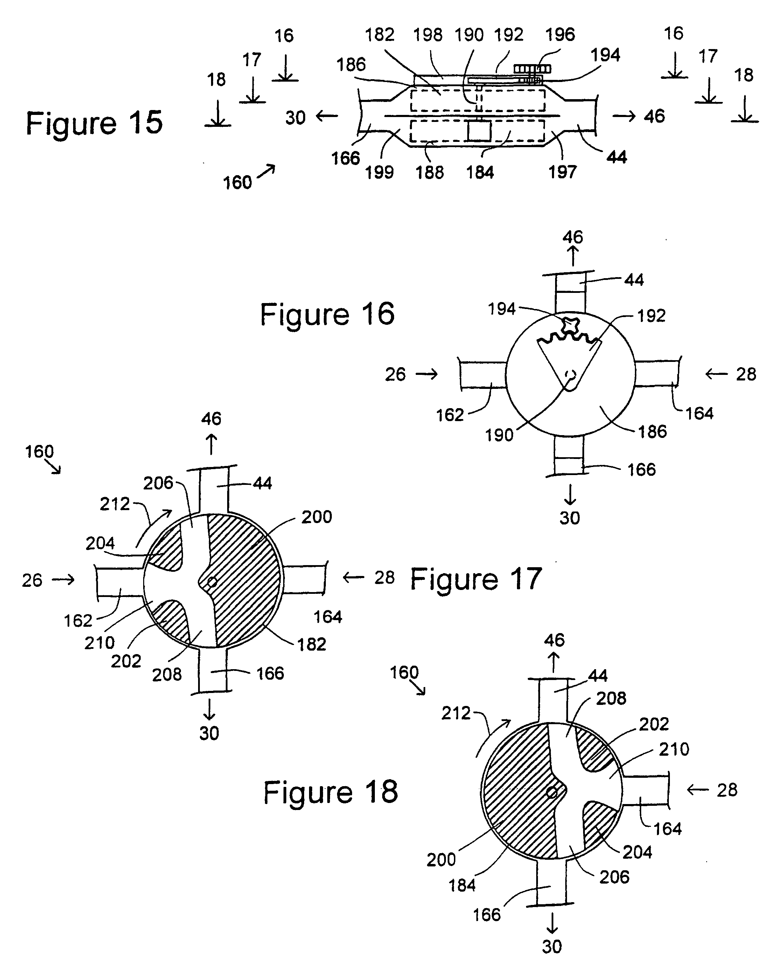

In this embodiment the outputs of syringes 12 and 14 are selectively distributed between mixing chamber 30 and waste chamber 46 by a flow-switching valve 160. Figure 13 illustrates a simple "either/or" embodiment of flow switching valve 160 wherein either reservoir 26 or reservoir 28 is connected with mixing chamber 30, while the output of the other reservoir, 28 or 26, is directed to the waste chamber 46. In Figure 15 a more complex embodiment of switching valve 160 is shown which provides for proportioning of the flows from reservoirs 26 and 28. Referring to Figure 12, syringes 12 and 14 communicate through respective conduits 162 and 164 with valve 160 which outputs through a conduit 166 to mixing chamber 30 and through conduit 44 to waste chamber 46. Employing the simple apparatus shown, wherein plungers 16, 18 and 20 are moved in harness by yoke 22, use of less than the full output of either syringe 12 or 14 means there is a balance of unneeded liquid discharged when yoke 22 is advanced. This balance is sent to waste chamber 46 by valve 160 to be discarded or re-used.

If desired, for example, because downstream perturbations are problematic, an optional flow restricter 168, and possibly also 170 (although this may not be necessary), can be provided in conduits 166 and 44 respectively, to serve a pressure isolating function providing a pressure zone on either downstream side of valve 160, when a flow proportioning embodiment is employed, to stabilize its operation. For balancing purposes, a third flow restricter 171 can be provided in conduit 29 if desired.

A further option is to provide a pressure-sensitive clog indicator 173 to provide a visual, and optionally an audio indication to the user of the occurrence of clogging of the applicator, as indicated by an increase in downstream flow resistance and of fluid pressure in the upstream conduits. One preferred location for such a clog indicator is in conduit 29 between reservoir 24 and mixing chamber 30, preferably downstream of flow restricter 171, if such is present, where it will be quickly responsive to flow interruptions in mixing chamber 30. Clog indicator 173 can comprise a spring-loaded visual indicator, or flag, which is tripped when a preset fluid pressure is detected in conduit 29, prompting the user to take suitable clearing action, e.g. to activate retrograde suctional clearing of mixing chamber 30 and cannula 32.

Flow restricters 168, 170 and 171 should have sufficient throat, or cross-sectional area, to provide satisfactory fluid delivery rates, without providing undue resistance that would make the applicator difficult to operate. Flow restricters 168 and 171 preferably have similar-sized throats to balance the fluid flows to chamber 30. Restricter 170 is preferably also of the same size to avoid the dumping of excess fluid into waste chamber 46 when abnormal back pressures occur. Suitable values of the flow restricter throat areas, in relation to the cross-sectional areas of conduits 29, 44 and 166, that will meet these criteria, will be apparent, or can readily be determined without undue experimentation. Throat areas of the order of 40 to 80 percent of the area of the respective conduit are contemplated as being effective. Such restricters 168,178 and 171 can of course also be employed in other embodiments of the invention described herein and should have cross-sectional areas proportional to the desired fluid flow rate in the conduits in which they are located.

Flow restricters 168, 170 can be helpful if back pressure from mixing chamber 30 or cannula 32, caused by residual solids deposits therein, forces an unintended proportion of reservoir liquids to waste chamber 46, diminishing the delivered proportion. In surgical applications, in particular, where the surgeon may be dispensing a pharmaceutical agent from one of syringes 12-14, consistency and predictability of device operation is important because the surgeon must know the precise dose administered. To alleviate this problem, flow restricter 168, and preferably also flow restricter 170, can be employed. Alternatively, thorough clearing of mixing chamber 30, and cannula 32, or employment of separate cannulas 70 -74 (Figure 10), with external mixing, may make such pressure control devices as flow restricters 168, 170 unnecessary.

Like two-way valve 40, valve 160 is manually operable, or can be coupled to be driven by a manually operable selector 172 through a coupling 173, to enable a desired liquid (Figure 13), or ratio of liquids (Figures 15-18) from reservoirs 26 and 28 to be manually selected by the user. The selector 48 shown in Figure 12 comprises a manually rotatable knob or dial, which could equally well be a lever or slide, which is positioned on the applicator to be externally accessible and incrementally movable to set or select the desired fluid or fluid ratio. Other mechanical or even electrical or electronic devices enabling a user to set flow-switching valve 160 will be apparent to those skilled in the art. Preferably, a scale 174 or other display provides a visual indication of the setting. The characteristics of flow switching valve 160 can be selected by those skilled in the art to provide a desired continuous range of mixing ratios, or a desired number of specific ratios, a desired response characteristic, i.e variation of mixing ratio, with mechanical setting, or other characteristics, as will be apparent to those skilled in the art.

The simplified "either/or" embodiment of valve 160 shown in Figure 13 comprises a rotatable valve member 176 having two fluid passages 178, 180, each turned through 90 degrees, to provide four ports spaced equi-distantly around the valve member 176. As valve member 176 rotates, fluid passages 178, 180 successively communicate with conduits 162 and 164 from reservoirs 26 and 28 respectively, and with conduits 166 and 44 leading to mixing chamber 30 and waste chamber 46 respectively.

Since fluid from either one or the other of reservoirs 26 and 28, but not from both, is supplied to mixing chamber 30, both reservoirs 26 and 28 should contain a thrombin agent or other catalyst for fibrinogen or other polymerization agent in reservoir 24 when using the Figure 13 embodiment of valve 160. Thus, reservoir 26 could contain a low concentration of thrombin while reservoir 28 contained a high concentration. Optionally reservoirs 26 and 28 could contain the same concentration of thrombin and one reservoir could also contain a therapeutic or conditioning agent, for example an antibiotic. Equally, the therapeutic agent could be associated with a different concentration of thrombin, if desired. Or a different therapeutic agent, serving a different purpose, could be combined with a desired concentration of thrombin in each reservoir. Other possibilities will be apparent from the teachings herein.

In a first position of valve member 176, as shown in Figure 13, the output from reservoir 28 is all directed to mixing chamber 13, through fluid passage 178, while the output from reservoir 26 all goes through fluid passage 180 to waste chamber 46. Rotating valve member 176 through 90 degrees (in either direction), reverses the flow whereby the output from reservoir 28 is all directed through fluid passage 180 to waste chamber 46 and all the output from reservoir 26 is directed through fluid passage 178 to mixing chamber 30. Intermediate positions are "off" positions. Obviously, each fluid passage 178 can subtend an angle other than 90 degrees, so long as they both subtend the same angle and conduits 162-166 and 44 are properly positioned to align.

Figure 14 illustrates a modified two-position selector 172 and scale 174 suitable for use with the embodiment of valve 160 shown in Figure 13 to effect and indicate, for example, selection of low thrombin or high thrombin, should such varied concentrations of fluid components be accommodated in reservoirs 26 and 28.

Referring to Figures 15-18, one possible flow-proportioning embodiment of valve 160 comprises a two-stage valve each stage of which is separately devoted to proportioning fluid from one or the other of reservoirs 26 or 28, between mixing chamber 30 and waste chamber 46. Each stage comprises a disk-shaped valve member 182 or 184 housed in a valve chamber 186 or 188. Valve chambers 186 and 188 are stacked one on top of the other in vertical alignment. Valve members 182 and 184 are fixedly mounted on a common shaft 190 which protrudes from the top of valve chamber 186 where it carries a section 192 of a large pinion gear. Gear section 192 is driven by a small pinion gear 194 with a substantial reducing ratio to provide a satisfactory response and feel to valve 160. A rotatable knurled disk 196 is mounted on pinion 194 externally of a cover 198 which houses gear section 192 and pinion 194. Rotation of disk 196, which may bear a setting indicator, positions valve members 182, 184, which move in tandem, to a desired setting, and can be effected by manual operation or may be driven by coupling 173. Thus, if it is suitably disposed, disk 196 may constitute selector 172.

Conduits 162 and 164 leading from reservoirs 26 and 28 communicate respectively with upper valve chamber 186 and lower valve chamber 188, while each of conduits 166 and 44 leading to mixing chamber 30 and to waste reservoir 46, respectively, has a Y-shaped split feed to valve 160 to serve both valve stages, referenced 197 and 199 in Figure 15. In the embodiment shown, conduits 44 and 162-166 are spaced at equal 90 degree intervals around valve 160. However, other non-perpendicular orientations can be used.

Referring to Figure 17, valve member 182 comprises three lands 200-204 which define a three-way fluid passage having branches 206-210 disposed on the left-hand side of valve member 182, referring to the position shown. Branches 206 and 208 terminate at the periphery of valve member 182 in ports which preferably correspond in size and shape with the cross sections of the registering conduits 44 and 166. To provide a linear relationship between the peripheral movement of valve member 182 and an increase or decrease in the area of registration of the branch passage ports with the conduits, as valve member 182 rotates in the clockwise direction indicated by arrow 212, it is preferred that the ports and conduits have a rectangular, more preferably, square shape where they register. If desired, the rectangular section of conduits 44 and 166 can merge to a more customary circular section away from valve 160.

Branches 206 and 208 are circumferentially staggered so that they communicate with their respective conduits 44 and 166 in sequence. Preferably any null point between such phases of communication which would be an "off" position, with no flow through the valve, is suitably marked on indicator 174. As shown in Figure 17, and referencing clockwise rotation of valve member 182, branch 206 has just begun to communicate with conduit 44, the area of registration is increasing and fluid flow to waste chamber 46 from reservoir 26 will increase if valve member 182 is further rotated. On the other hand, branch 208 is in almost full communication with conduit 166, the area of registration is decreasing and further rotation of valve member 182 will result in a decreased flow of fluid from reservoir 26 to mixing chamber 30. For consistency of operation and predictability of the composition of sealant output 34, pressure balancing is desirable and it is therefore preferred that the sum of the areas of registration of branches 206 and 208 with their respective conduits 44 and 166 remain constant, and be equal, for example to the area of each conduit 44 or 166 at the valve 160.

Conduit 162 should also preferably terminate in a rectangular, more preferably square, section while branch 210, which registers with conduit 162, should terminate in a port with a rectangular shape having a circumferential width equal to the sum of the circumferential widths of branches 206 and 208 so that branch 210 is in full communication with conduit 162 during the whole time that branch 206 or branch 208 communicates with its respective conduit 44 or 166.

Referring now to Figure 18, valve member 184 in the lower stage of valve 160 is similar to valve member 182 but is rotated to be 180 degrees out of phase with valve 182, as shown in the figure. Valve member 184 thus provides a similar function of distributing fluid flow from reservoir 28 between mixing chamber 30 and waste chamber 46 but with opposite timing from the distribution of fluid from reservoir 26 in the upper stage of valve 160.

Accordingly, noting that valve members 182 and 184 are coupled together to move in tandem, in the position where valve member 182 in the upper stage directs all fluid flow from reservoir 26 to mixing chamber 30, valve member 184 in the lower stage will direct all fluid flow from reservoir 28 to waste chamber 46. Similarly, in the position where valve member 182 in the upper stage directs all fluid flow from reservoir 26 to waste chamber 46, valve member 184 in the lower stage will direct all fluid flow from reservoir 28 to mixing chamber 30.

In the positions shown in Figures 17 and 18, a large flow from reservoir 26 in the upper stage joins with a small flow from reservoir 28, in split feed 199, and goes to mixing volume 30. At the same time, a small quantity of fluid from reservoir 26, the balance, joins with a large quantity of fluid from reservoir 28, in split feed 197, and goes to waste chamber 46. The proportioning characteristics of valve 160 in other intermediate positions between the extremes will be apparent to those skilled in the art. Thus, as valve member 182 in the upper stage of valve 160 rotates in the clockwise direction of arrow 212 from the position shown in Figure 17, the proportion of the fluid flow from reservoir 26 going to waste chamber 46 increases, and the proportion sent to mixing chamber 30 diminishes. As valve member 184 in the lower stage rotates with valve member 182, from the position shown in Figure 18, the proportion of the fluid flow from reservoir 28 going to waste chamber 46 diminishes, and the proportion sent to mixing chamber 30 increases.

Selector 172 (which could be dial 96, if desired) can thus provide the user with a continuous range of variation of proportions of the two fluid sealants from reservoirs 26 and 28 to deliver to mixing chamber 30. Alternatively, it could lock into a small number of set positions in the range. If desired, an "off'position can be provided wherein each conduit 44 and 162-166 is closed by a respective land 200-204 on the valve member 182 or 184, and no fluid can flow through valve 160.
There is no particular limit to the number of possible combinations of fluid components that can be incorporated into syringes 10-14 and selectively dispensed by the user, in a variable manner, by choosing an appropriate setting of valve 160, subject to providing a polymerizable agent and a catalytic agent, and to keeping these agents apart until they enter mixing chamber 30 or are dispensed. The proportioning feature of the embodiment of valve 160 shown in Figures 15-18 can be utilized to provide quantitative variations in sealant output 34, for example by varying the concentration of one of the fluid components or qualitative variations by selecting the components included in sealant output 34.

For example, syringe 10 can contain fibrinogen, syringe 12 can contain a low concentration of thrombin, or a thrombin diluent such as a calcium chloride solution, and the third syringe, in this case syringe 14, can contain a high concentration of thrombin. Selector 172 can be operated by the user to select any desired concentration of thrombin between (and including) the concentrations in syringes 12 and 14. Proportioning valve 160 controls the output, adding a desired amount of thrombin and discharging the balance to waste chamber 46.

In an alternative example, the third syringe 14 can have a desired concentration of a therapeutic or other auxiliary agent which is added in varying concentrations, a desired concentration of thrombin being provided in syringe 12. To avoid a reduction of the thrombin concentration in the sealant output 34, as higher proportions of the auxiliary agent are added from syringe 14 and, therefore, smaller proportions of thrombin are added from syringe 12, syringe 14 may also include an equal concentration of thrombin to that in syringe 12, if desired. Alternatively, syringe 14 may contain an adjusted concentration of thrombin along with the auxiliary agent, if such adjusted concentration is appropriate in the presence of the auxiliary agent.

While the illustrated flow proportioning embodiment of valve 160, as shown in Figures 15-18 contemplates that syringes 12 and 14 will have substantially equal output flow rates, from their respective reservoirs 26 and 28, and therefore provides, or prefers, equally dimensioned flow paths in the upper stage (from reservoir 26) and in the lower stage (from reservoir 28), it will be understood that should a smaller section syringe, such as is shown in Figures 1 and 4A be employed, the flow passages in the appropriate stage of valve 160 can be dimensioned accordingly.

The range of settings provided by valve 160 can vary widely being, for example, at one extreme, from ten parts, or more, of diluent or other agent from reservoir 26 to one part of thrombin, or other agent, from reservoir 28, to provide a very dilute activator and a slow-setting relatively rigid gel. At the other extreme, zero diluent is employed, to utilize a maximum thrombin concentration and provide a fast-setting sealant. Preferably, the range is such that a substantial portion of the movement of selector 172 provides ratios near to a 1:1 balance of diluent to thrombin for example, ratios in the range of from 0.5:1 to 2:1 parts diluent per part thrombin.
Many possible variations and modifications will be apparent those skilled in the art as coming within the scope of the invention. For example reservoirs 24, 26 and 28 although depicted as being provided within syringes 10, 12 and 14, can be provided by any suitable fluid retaining plenum of readily variable volume, for example compressible bladders, squeezable tubes or other fluid-retaining vessels from which controlled volumes of fluid may be expelled by application of a suitable force applied by an internal or external fluid discharge member such as a plunger or lever. Alternatively, the reservoirs may be of fixed volume and their contents can be expelled pneumatically, for example, by admitting compressed air or other gas to the reservoir.

Similarly, equivalent biological or other fluid components may be used in place of fibrinogen and thrombin. The addition of a third sealant agent source, for convenience only here referenced as syringe 14, adds a new dimension of capabilities and options to the fibrin sealant art and to the choices that can be made available to a surgeon, or other operator. In addition to the foregoing variations in strength and setting parameters of the sealant described hereinabove, many other desirable results can be obtained by deploying other useful agents from a third syringe. For example, the sealant output may be used as a carrier for therapeutic or nutritive agents, for example growth factors or cell cultures that can aid in wound management, enabling such desirable agents to be delivered precisely to the location where they are required, namely at the wound interface. Also, the sealant may be conditioned or reinforced by deploying materials such as collagen or elastin. A tissue work surface may receive therapeutic treatment by deploying a drug or medication, for example an antibiotic, or the site of application may be used as an entry point to the body and a drug or other therapeutic agent may be incorporated in a biologically compatible vector, for example a liposome, targeted to a remote organ.

Other options are to apply local anaesthetics, indicators or healing aids via reservoir 26, or its equivalent, for example an external reservoir or other source feeding the agent to the applicator via a supply line.

It will be understood that the principles of the invention can be applied to sealant applicators and methods employing not just three but even four or more fluid component sources for particular purposes related to the application or the functionality of the sealant, or of auxiliary agents delivered with the sealant. However, undue multiplicity of the fluid component sources may become cumbersome.

Whereas the invention has been described in the context of a hand-held applicator suited to the needs of the surgeon in the operating room, and manual operation and actuation of the applicator have been primarily envisaged, it is nevertheless contemplated that for certain, at present unforeseen, special purposes the applicator of the invention could be remote-controlled, or robot-controlled or computer-controlled, with functions described herein as being manually performed being effected by equivalent mechanical, hydraulic or pneumatic actuators under suitable automated control.

If desired, sealant output 34, or the fluid components output from cannula 70 and 72, or 70-74, may be gas-entrained for spray deposition on the work surface, as taught in copending Epstein patent application number 60/083,854.

While illustrative embodiments of the invention have been described above, it is, of course, understood that various modifications will be apparent to those of ordinary skill in the art. Many such modifications are contemplated as being within the scope of the invention, as defined in the appended claims.

## Claims

1. A variable output handheld fluid agent applicator (10) for dispensing a desired multi-component fluid agent to a site of use, the applicator comprising:
(i) at least first, second and third fluid component sources (24, 26,28) for holding at least first, second and third fluid components;
(ii) a dispensing tip for delivering the first fluid component and at least a portion of the second fluid component and/or at least a portion of the third fluid component to the site of use;
(iii) a manually actuated delivery system communicating with the fluid component sources for delivering the fluid components to the dispensing tip;
(iv) a waste chamber (46); and
(v) a flow-switching valve (160) coupled to the delivery system and adapted for selectively distributing an output of the second and third component sources between the dispensing tip and the waste chamber so as to provide a multi-component fluid agent having a desired composition,
wherein the delivery system delivers the desired multi-component fluid agent having a substantially constant composition.

2. An applicator according to claim 1, wherein the flow-switching valve (160) is further adapted to direct the whole of the output of the second component source (26) to the dispensing tip and to direct the whole of the output of the third component source (28) to the waste chamber (46).

3. An applicator according to claim 1, wherein the flow-switching valve (160) is further adapted to proportion the outputs of the second and third fluid component sources (26, 28) between the dispensing tip and the waste chamber (46).

4. An applicator according to any one of the preceding claims, further comprising a mixing chamber (30) adapted for mixing the fluid components prior to delivery to the dispensing tip.

5. An applicator according to claim 4, further comprising a first flow restrictor (168) positioned between the mixing chamber (30) and the third fluid component source (28).

6. An applicator according to claim 4 or 5, further comprising a second flow restrictor (170) positioned between the waste chamber (46) and the third fluid component source (28).

7. An applicator according to any one of the preceding claims, wherein the first fluid component comprises a polymerisable agent and the second fluid component comprises a polymerisation catalyst.

8. An applicator according to any one of claims 3 to 6, wherein the first fluid component comprises a polymerisable agent, the second fluid component comprises a polymerisation activation agent and the third component comprises a diluent for the activation agent, and wherein the flow-switching valve (160) is adapted to direct at least a portion of the polymerisation activation agent and at least a portion of the diluent to the dispensing tip so as to provide desired multi-component fluid agents of varying activity levels.

9. An applicator according to any one of claims 3 to 6, wherein the first fluid component comprises a polymerisable agent, the second fluid component comprises a polymerisation activation agent and the third component comprises a diluent for the activation agent, and wherein the flow-switching valve (160) is adapted to direct constant proportions of the polymerisable agent and diluent, as well as varying proportions of the polymerisation activation agent, to the dispensing tip so as to provide desired multi-component fluid agents having varying setting times.

10. An applicator according to any one of claims 3 to 6, wherein the first fluid component comprises a polymerisable agent, the second fluid component comprises a first polymerisation activation agent having a first concentration and the third component comprises a second polymerisation activation agent having a second concentration different to the first, and wherein the flow-switching valve (160) is adapted to direct at least a portion of one of the polymerisation activation agents to the dispensing tip.

11. An applicator according to any one of claims 1 to 6, wherein the first fluid component comprises fibrinogen and the second and the third fluid components comprise thrombin, and wherein the flow-switching valve (160) is adapted to direct fibrin sealants having selected thrombin concentrations to the dispensing tip.

12. An applicator according to any one of claims 1 to 6, wherein the first fluid component comprises a polymerisable agent, the second fluid component comprises a polymerisation activation agent and the third component comprises a conditioning or a therapeutic agent.

13. An applicator according to any one of the preceding claims, wherein the flow-switching valve (160) is operable manually.

14. An applicator according to any one of the preceding claims, wherein the flow-switching valve (160) is driven by a manually operable selector (172).

15. An applicator according to claim 14, wherein the selector (172) comprises a rotatable dial, a pivotable lever, a depressable button or a movable slide.

16. An applicator according to any one of the preceding claims, which comprises first, second and third fluid component sources (24, 26, 28).

17. An applicator according to claim 16, further comprising three plungers (16, 18, 20) to discharge the fluid components from the first, second and third fluid component sources (24, 26, 28) and a yoke (22) to drive the plungers.

## Patentansprüche

1. Fluidwirkstoff-Handapplikator (10) mit veränderbarer Ausgabe zum Abgeben eines gewünschten Mehrkomponenten-Fluidwirkstoffs an eine Verwendungsstelle, wobei der Applikator folgendes aufweist:
(i) mindestens eine erste, eine zweite und eine dritte Fluidkomponentenquelle (24, 26, 28) zum Halten von mindestens einer ersten, einer zweiten und einer dritten Fluidkomponente;
(ii) eine Abgabespitze zum Zuführen der ersten Fluidkomponente und mindestens eines Anteils der zweiten Fluidkomponente und/oder mindestens eines Anteils der dritten Fluidkomponente an die Verwendungsstelle;
(iii) ein handbetätigtes Zuführsystem, das mit den Fluidkomponentenquellen in Verbindung steht, zum Zuführen der Fluidkomponenten an die Abgabespitze;
(iv) eine Entsorgungskammer (46); und
(v) ein Durchflußumschaltventil (160), das mit dem Zuführsystem gekoppelt und so ausgebildet ist, daß es eine Fördermenge von der zweiten und der dritten Komponentenquelle zwischen der Abgabespitze und der Entsorgangskammer selektiv verteilt, um einen Mehrkomponenten-Fluidwirkstoff bereitzustellen, der eine gewünschte Zusammensetzung hat,
wobei das Zuführsystem den gewünschten Mehrkomponenten-Fluidwirkstoff, der eine im wesentlichen konstante Zusammensetzung hat, zuführt.

2. Applikator nach Anspruch 1, wobei das Durchflußumschaltventil (160) fernet so ausgebildet ist, daß es die gesamte Fördermenge von der zweiten Komponentenquelle (26) zu der Abgabespitze leitet und die gesamte Fördermenge von der dritten Komponentenquelle (28) zu der Entsorgungskammer (46) leitet.

3. Applikator nach Anspruch 1, wobei das Durchflußumschaltventil (160) ferner so ausgebildet ist, daß es die Fördermengen von der zweiten und der dritten Fluidkomponentenquelle (26, 28) zwischen der Abgabespitze und der Entsorgungskammer (46) zuteilt.

4. Applikator nach einem der vorhergehenden Ansprüche, der ferner eine Mischkammer (30) aufweist, die so ausgebildet ist, daß sie die Fluidkomponenten vor dem Zuführen an die Abgabespitze vermischt.

5. Applikator nach Anspruch 4, der ferner einen ersten Durchflußbegrenzer (168) aufweist, der zwischen der Mischkammer (30) und der dritten Fluidkomponentenquelle (28) positioniert ist.

6. Applikator nach Anspruch 4 oder 5, der ferner einen zweiten Durchflußbegrenzer (170) aufweist, der zwischen der Entsorgungskammer (46) und der dritten Fluidkomponentenquelle (28) positioniert ist.

7. Applikator nach einem der vorhergehenden Ansprüche, wobei die erste Fluidkomponente ein polymerisierbares Mittel aufweist und die zweite Fluidkomponente einen Polymerisationskatalysator aufweist.

8. Applikator nach einem der Ansprüche 3 bis 6, wobei die erste Fluidkomponente ein polymerisierbares Mittel aufweist, die zweite Fluidkomponente ein Polymerisationsaktivierungsmittel aufweist und die dritte Komponente ein Verdünnungsmittel für das Aktivierungsmittel aufweist, und wobei das Durchflußumschaltventil (160) so ausgebildet ist, daß es mindestens einen Anteil des Polymerisationsaktivierungsmittels und mindestens einen Anteil des Verdünnungsmittels zu der Abgabespitze leitet, um die gewünschten Mehrkomponenten-Fluidwirkstoffe mit veränderbaren Aktivitätsgraden bereitzustellen.

9. Applikator nach einem der Ansprüche 3 bis 6, wobei die erste Fluidkomponente ein polymerisierbares Mittel aufweist, die zweite Fluidkomponente ein Polymerisationsaktivierungsmittel aufweist und die dritte Komponente ein Verdünnungsmittel für das Aktivierungsmittel aufweist, und wobei das Durchflußumschaltventil (160) so ausgebildet ist, daß es konstante Anteile des polymerisierbaren Mittels und des Verdünnungsmittels sowie veränderbare Anteile des Polymerisationsaktivierungsmittels zu der Abgabespitze leitet, um gewünschte Mehrkomponenten-Fluidwirkstoffe bereitzustellen, die veränderbare Abbindezeiten haben.

10. Applikator nach einem der Ansprüche 3 bis 6, wobei die erste Fluidkomponente ein polymerisierbares Mittel aufweist, die zweite Fluidkomponente ein erstes Polymerisationsaktivierungsmittel aufweist, das eine erste Konzentration hat, und die dritte Komponente ein zweites Polymerisationsaktivierungsmittel aufweist, das eine zweite Konzentration hat, die von der ersten verschieden ist, und wobei das Durchflußumschaltventil (160) so ausgebildet ist, daß es mindestens einen Anteil von einem von den Polymerisationsaktivierungsmitteln zu der Abgabespitze leitet.

11. Applikator nach einem der Ansprüche 1 bis 6, wobei die erste Fluidkomponente Fibrinogen aufweist und die zweite und die dritte Fluidkomponente Thrombin aufweisen, und wobei das Durchflußumschaltventil (160) so ausgebildet ist, daß es Fibrindichtungsmittel, die ausgewählte Thrombinkonzentrationen haben, zu der Abgabespitze leitet.

12. Applikator nach einem der Ansprüche 1 bis 6, wobei die erste Fluidkomponente ein polymerisierbares Mittel aufweist, die zweite Fluidkomponente ein Polymerisationsaktivierungsmittel aufweist und die dritte Komponente ein Konditionierungs- oder ein therapeutisches Mittel aufweist.

13. Applikator nach einem der vorhergehenden Ansprüche, wobei das Durchflußumschaltventil (160) handbetätigbar ist.

14. Applikator nach einem der vorhergehenden Ansprüche, wobei das Durchflußumschaltventil (160) von einer handbetätigbaren Wähleinrichtung (172) angetrieben wird.

15. Applikator nach Anspruch 14, wobei die Wähleinrichtung (172) eine drehbare Wählscheibe, einen schwenkbaren Hebel, eine niederdrückbare Taste oder einen bewegbaren Schieber aufweist.

16. Applikator nach einem der vorhergehenden Ansprüche, der eine erste, eine zweite und eine dritte Fluidkomponentenquelle (24, 26, 28) aufweist.

17. Applikator nach Anspruch 16, der ferner aufweist: drei Kolben (16, 18, 20), um die Fluidkomponenten aus der ersten, der zweiten und der dritten Fluidkomponentenquelle (24, 26, 28) abzugeben, und ein Joch (22), um die Kolben anzutreiben.

## Revendications

1. Applicateur d'agent fluide portable à sortie variable (10) permettant de distribuer un agent fluide à multicomposants souhaité sur un site d'utilisation, l'applicateur comprenant :
(i) au moins des première, deuxième et troisième sources de composants fluides (24, 26, 28) destinées à contenir au moins des premier, deuxième et troisième composants fluides ;
(ii) une buse distributrice destinée à délivrer le premier composant fluide et au moins une partie du deuxième composant fluide et/ou au moins une partie du troisième composant fluide sur le site d'utilisation ;
(iii) un système de délivrance à action manuelle communiquant avec les sources de composants fluides pour délivrer les composants fluides à la buse distributrice ;
(iv) une chambre d'excédent (46) ; et
(v) une vanne de sélection de débit (160) couplée au système de délivrance et adaptée pour répartir sélectivement une sortie des deuxième et troisième sources de composants entre la buse distributrice et la chambre d'excédent de façon à produire un agent fluide à multicomposants ayant une composition souhaitée,
dans lequel le système de délivrance délivre l'agent fluide à multicomposants souhaité ayant une composition sensiblement constante.

2. Applicateur selon la revendication 1, dans lequel la vanne de sélection de débit (160) est en outre adaptée pour diriger l'ensemble de la sortie de la deuxième source de composant (26) vers la buse distributrice et pour diriger l'ensemble de la sortie de la troisième source de composant (28) vers la chambre d'excédent.

3. Applicateur selon la revendication 1, dans lequel la vanne de sélection de débit (160) est en outre adaptée pour répartir de façon proportionnelle les sorties des deuxième et troisième sources de composants fluides (26, 28) entre la buse distributrice et la chambre d'excédent (46).

4. Applicateur selon l'une quelconque des revendications précédentes, comprenant en outre une chambre de mélange (30) adaptée pour mélanger les composants fluides avant leur délivrance à la buse distributrice.

5. Applicateur selon la revendication 4, comprenant en outre un premier limiteur de débit (168) placé entre la chambre de mélange (30) et la troisième source de composant fluide (28).

6. Applicateur selon la revendication 4 ou 5, comprenant en outre un deuxième limiteur de débit (170) placé entre la chambre d'excédent (46) et la troisième source de composant fluide (28).

7. Applicateur selon l'une quelconque des revendications précédentes, dans lequel le premier composant fluide comprend un agent polymérisable et le deuxième composant comprend un catalyseur de polymérisation.

8. Applicateur selon l'une quelconque des revendications 3 à 6, dans lequel le premier composant fluide comprend un agent polymérisable, le deuxième composant fluide comprend un agent d'activation de polymérisation et le troisième composant comprend un diluant pour l'agent d'activation, et dans lequel la vanne de sélection de débit (160) est adaptée pour diriger au moins une partie de l'agent d'activation de polymérisation et au moins une partie du diluant vers la buse distributrice de façon à produire des agents fluides à multicomposants souhaités présentant des niveaux d'activité variables.

9. Applicateur selon l'une quelconque des revendications 3 à 6, dans lequel le premier composant fluide comprend un agent polymérisable, le deuxième composant fluide comprend un agent d'activation de polymérisation et le troisième composant comprend un diluant pour l'agent d'activation, et dans lequel la vanne de sélection de débit (160) est adaptée pour diriger des proportions constantes de l'agent polymérisable et du diluant ainsi que des proportions variables de l'agent d'activation de polymérisation vers la buse distributrice de façon à produire des agents fluides à multicomposants souhaités ayant des temps de prise variables.

10. , Applicateur selon l'une quelconque des revendications 3 à 6, dans lequel le premier composant fluide comprend un agent polymérisable, le deuxième composant fluide comprend un premier agent d'activation de polymérisation ayant une première concentration et le troisième composant comprend un deuxième agent d'activation de polymérisation ayant une deuxième concentration différente de la première, et dans lequel la vanne de sélection de débit (160) est adaptée pour diriger au moins une partie de l'un des agents d'activation de polymérisation vers la buse distributrice.

11. Applicateur selon l'une quelconque des revendications 1 à 6, dans lequel le premier composant fluide comprend du fibrinogène et les deuxième et troisième composants fluides comprennent de la thrombine, et dans lequel la vanne de sélection de débit (160) est adaptée pour diriger vers la buse distributrice des colles de fibrine ayant des concentrations de thrombine choisies.

12. Applicateur selon l'une quelconque des revendications 1 à 6, dans lequel le premier composant fluide comprend un agent polymérisable, le deuxième composant fluide comprend un agent d'activation de polymérisation et le troisième composant comprend un agent de conditionnement ou un agent thérapeutique.

13. Applicateur selon l'une quelconque des revendications précédentes, dans lequel la vanne de sélection de débit (160) peut être actionnée à la main.

14. Applicateur selon l'une quelconque des revendications précédentes, dans lequel la vanne de sélection de débit (160) est commandée par un sélecteur à action manuelle (172).

15. Applicateur selon la revendication 14, dans lequel le sélecteur (172) comprend un cadran rotatif, un levier pivotant, un bouton poussoir ou un curseur mobile.

16. Applicateur selon l'une quelconque des revendications précédentes, qui comprend des première, deuxième et troisième sources de composants fluides (24, 26, 28).

17. Applicateur selon la revendication 16, comprenant en outre trois pistons (16, 18, 20) pour expulser les composants fluides des première, deuxième et troisième sources de composants fluides (24, 26, 28) et une rampe de couplage (22) pour actionner les pistons.
